# EUROPEAN PATENT APPLICATION

(11) **EP 4 389 755 A1**
(43) Date of publication of application: **26.06.2024**
(21) Application number: 22858505.5
(22) Date of filing: 18.08.2022
(51) Int. Cl.: C07F 5/02, C09K 11/06, C09B 23/04, G01N 33/533, G01N 21/64

(54) **FLUORESCENT COMPOUND AND FLUORESCENCE-LABELED BIOSUBSTANCE OBTAINED USING SAME**

(30) Priority: 18.08.2021 JP 2021133510
(71) Applicant: FUJIFILM Corporation, Tokyo 106-8620 (JP)
(72) Inventor: WATANABE, Kousuke, Ashigarakami-gun, Kanagawa 258-8577 (JP)
(74) Representative: HGF
(86) International application number: PCT/JP2022/031169
(87) International publication number: WO 2023/022187

(57) **Abstract**

There are provided a fluorescent compound represented by General Formula (1) and a fluorescently labeled biological substance using the fluorescent compound.

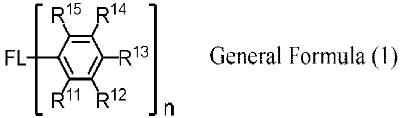

In the formula, FL indicates an n-valent phosphor moiety, and n is an integer of 1 or more.

R¹¹ to R¹⁵ represent a hydrogen atom or a substituent. However, at least one combination of the combination of R¹¹ and R¹² or the combination of R¹² and R¹³ is a combination in which one is -SO₃⁻X⁺ and the other is a substituent. X⁺ represents a hydrogen ion or a metal ion.

## Description

### BACKGROUND OF THE INVENTION

### 1. Field of the Invention

The present invention relates to a fluorescent compound and a fluorescently labeled biological substance using the fluorescent compound.

### 2. Description of the Related Art

In order to observe in vivo changes in response to various stimuli (diseases, environmental changes, and the like), fluorescently labeled biological substances obtained by labeling a biological molecule (an antibody or the like) having a binding property to a target substance to be detected, with a fluorescent compound (a fluorescent dye), are often used.

For example, also in Western blotting (hereinafter, also abbreviated as WB) that detects a specific protein from a protein mixture, a fluorescence method in which the presence or absence or the abundance of the specific protein is detected using a fluorescently labeled antibody having a binding property to this protein is used.

In addition, in bioimaging technology for analyzing the dynamics and functions of biological molecules, cells, tissues, and the like in a living body, in vivo fluorescence imaging in which a specific portion of a living body visualized by fluorescence labeling is observed is used as one of the techniques for the living body observation.

In the fluorescence labeling, an organic fluorescent dye is generally used.

However, since most of the organic dyes exhibiting fluorescence, such as a dipyrromethene dye, a cyanine dye, and a rhodamine dye, have an aromatic chromophore having high planarity, an interaction between the dyes easily occurs, and as a result, a decrease in the fluorescence intensity due to an interaction such as self-association between the dyes easily occurs.

In addition, the organic fluorescent dye has low light resistance and deteriorates by irradiation with excitation light, and thus the observation of the living body of interest may not be sufficiently carried out.

A dipyrromethene boron complex dye, which is one kind of organic fluorescent dye, is known as a fluorescent dye that has a high quantum yield and exhibits sharp luminescence characteristics, and the research on the application thereof to fluorescence labeling is underway.

For example, JP2019-172826A describes a dipyrromethene boron complex dye having an aryl group or a heteroaryl group in at least two sites of the 2-position, the 4-position, and the 6-position of a dipyrromethene skeleton and describes that the dipyrromethene boron complex dye suppresses the dye decomposition due to light irradiation and realizes high light resistance. In addition, WO2021/100814A describes a dipyrromethene boron complex dye having at least one alkyl group bonded to a boron atom and further having a specific hydrophilic group at a specific position of a dipyrromethene skeleton and describes that the dipyrromethene boron complex dye suppresses the dye decomposition due to light irradiation and realizes high light resistance while maintaining high water solubility.

### SUMMARY OF THE INVENTION

It is required that a dye that is used for fluorescence labeling has high hydrophilicity, exhibits an excellent fluorescence intensity, and exhibits excellent light resistance as a compound or a fluorescently labeled biological substance. However, as a result of studies by the inventors of the present invention, it was found that the fluorescent dye described in JP2019-172826A does not have sufficient characteristics in light resistance, and thus there is room for improvement, and it was found that the fluorescent dye described in WO2021/100814A does not have sufficient characteristics in the molar absorption coefficient does not have sufficient characteristics, there is room for improvement in terms of fluorescence intensity, and the fluorescent dye and an obtained fluorescently labeled biological substance do not have sufficient characteristics in light resistance, and thus there is room for improvement.

In addition, the present invention is not limited to the dipyrromethene boron complex dye as described in JP2019-172826A, WO2021/100814A, or the like, and it is important to search for a chemical structure that has high hydrophilicity and exhibits an excellent fluorescence intensity over the entire organic fluorescent dye and can exhibit excellent light resistance as a compound or a biological substance after being labeled.

An object of the present invention is to provide a fluorescent compound that has high water solubility required for fluorescence labeling, has a high molar absorption coefficient and a high fluorescence quantum yield, has an excellent fluorescence intensity (brightness), and can exhibit excellent light resistance as a compound or a fluorescently labeled biological substance. In addition, another object of the present invention is to provide a fluorescently labeled biological substance obtained by bonding the fluorescent compound to a biological substance.

That is, the above objects of the present invention have been achieved by the following means.
[1] A fluorescent compound represented by General Formula (1), 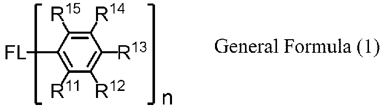
   in the formula, FL indicates a phosphor moiety and n is an integer of 1 or more,
   R¹¹ to R¹⁵ represent a hydrogen atom or a substituent,
   provided that at least one combination of a combination of R¹¹ and R¹² or a combination of R¹² and R¹³ is a combination in which one is -SO₃⁻X⁺ and the other is a substituent, and
   X⁺ represents a hydrogen ion or a metal ion.
[2] The fluorescent compound according to [1], in which the at least one combination of the combination of R¹¹ and R¹² or the combination of R¹² and R¹³ is a combination in which one is -SO₃⁻X⁺ and the other is a hydroxy group, an alkoxy group, an aryloxy group, or a heteroaryloxy group.
[3] The fluorescent compound according to [1] or [2], in which the fluorescent compound is represented by General Formula (2), 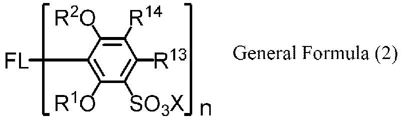
   in the formula, R¹ and R² represent a hydrogen atom, an alkyl group, an aryl group, or a heteroaryl group, and
   FL, R¹³, R¹⁴, X⁺, and n respectively have the same meanings as FL, R¹³, R¹⁴, X⁺, and n described above.
[4] The fluorescent compound according to [3], in which R¹ and R² described above are an alkyl group, an aryl group, or a heteroaryl group.
[5] The fluorescent compound according to [3] or [4], in which R¹ and R² described above are an alkyl group having an alkylene glycol structure.
[6] The fluorescent compound according to any one of [1] to [5], in which the FL is a structural moiety consisting of a dipyrromethene dye, a pyrrolopyrrole dye, a pyrene dye, a coumarin dye, or a cyanine dye.
[7] The fluorescent compound according to [6], in which the FL is a structural moiety consisting of a dipyrromethene boron complex dye.
[8] The fluorescent compound according to [7], in which the dipyrromethene boron complex dye is represented by General Formula (6), 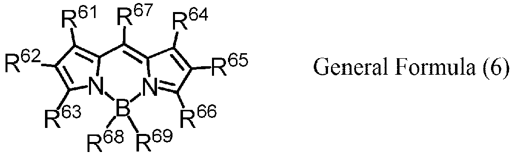
   in the formula, R⁶¹ to R⁶⁷ represent a hydrogen atom or a substituent,
   R⁶⁸ and R⁶⁹ represent a hydrogen atom, a hydroxy group, a halogen atom, an alkoxy group, an alkyl group, an aryl group, -C≡CR^{b}, or a cyano group,
   R^{b} represents a hydrogen atom, an alkyl group, or an aryl group, and
   in a dye represented by the formula, n pieces of hydrogen atoms are removed from any one of R⁶¹ to R⁶⁹ so that the FL is n-valent.
[9] The fluorescent compound according to [8], in which R⁶³ and R⁶⁶ described above are an alkyl group or an aryl group.
[10] The fluorescent compound according to [8], in which the dipyrromethene boron complex dye is represented by General Formula (7), 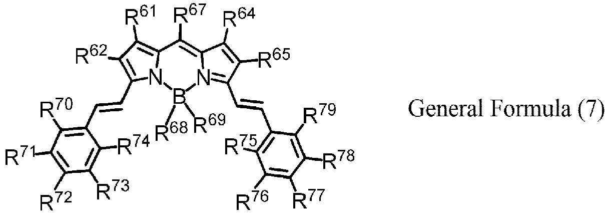
   in the formula, R⁶¹, R⁶², R⁶⁴, R⁶⁵, and R⁶⁷ to R⁶⁹ respectively have the same meanings as R⁶¹, R⁶², R⁶⁴, R⁶⁵, and R⁶⁷ to R⁶⁹ described above,
   R⁷⁰ to R⁷⁹ represent a hydrogen atom or a substituent, and
   in a dye represented by the formula, n pieces of hydrogen atoms are removed from any one of R⁶¹, R⁶², R⁶⁴, R⁶⁵, R⁶⁷ to R⁶⁹, or R⁷⁰ to R⁷⁹ so that the FL is n-valent.
[11] A fluorescently labeled biological substance that is obtained by bonding the fluorescent compound according to any one of [1] to [10] to a biological substance.
[12] The fluorescently labeled biological substance according to [11], in which the biological substance is any one of a protein, an amino acid, a nucleic acid, a sugar chain, or a lipid.
[13] The fluorescently labeled biological substance according to [11] or [12], in which the bonding of the fluorescent compound to the biological substance is a bonding formed by any one of the following i) to v),
   i) a non-covalent bond or covalent bond between peptides,
   ii) Van der Waals interaction between a long-chain alkyl group in a compound and a lipid bilayer or lipid in a biological substance,
   iii) an amide bond formed by reacting an N-hydroxysuccinimide ester in a compound with an amino group in a biological substance,
   iv) a thioether bond formed by reacting a maleimide group in a compound with a sulfanyl group in a biological substance, and
   v) a bond associated with a formation of a triazole ring, which is formed by a Click reaction between an azide group in a compound and an acetylene group in a biological substance, or between an acetylene group in a compound and an azide group in a biological substance.

The fluorescent compound according to the aspect of the present invention makes it possible to obtain a fluorescent compound that has high water solubility required for fluorescence labeling, has a high molar absorption coefficient and a high fluorescence quantum yield, has excellent brightness, and has excellent light resistance, where the fluorescent compound is a fluorescently labeled biological substance having excellent light resistance. In addition, the fluorescently labeled biological substance according to the aspect of the present invention exhibits excellent light resistance.

### DESCRIPTION OF THE PREFERRED EMBODIMENTS

In the present invention, in a case where there are a plurality of substituents or linking groups represented by a specific symbol or formula (hereinafter, referred to as substituents or the like), or in a case where a plurality of substituents or the like are defined at the same time, the substituents or the like may be the same or different from each other, unless otherwise specified. The same applies to the regulation of the number of substituents or the like. In addition, in a case where a plurality of substituents or the like come close to each other (particularly in a case where they are adjacent to each other), they may be linked to each other to form a ring, unless otherwise specified. In addition, in a case where a ring is formed, an unsaturated ring or an aromatic ring may be formed by removing a hydrogen atom. In addition, unless otherwise specified, rings such as an alicyclic ring, an aromatic ring, and a heterocyclic ring may be fused to form a fused ring.

In the present invention, in a case where an E type and a Z type of a double bond are present in a molecule, any one of the E type or the Z type, or a mixture thereof may be used unless otherwise specified. In addition, in a case where a compound has diastereomers and enantiomers, any one of the diastereomers or the enantiomers may be used, or a mixture thereof may be used unless otherwise specified.

In the present invention, the denotation of a compound or substituent is meant to include not only the compound itself but also a salt thereof, and an ion thereof. For example, the carboxy group, the sulfo group, and the phosphono group (-P(=O)(OH)₂) may have an ionic structure by a hydrogen ion being dissociated therefrom, or they may have a salt structure. That is, in the present invention, the "carboxy group" is meant to include an ion or salt of a carboxylic acid, the "sulfo group" is meant to include an ion or salt of a sulfonic acid, and the "phosphono group" is meant to include an ion or salt of a phosphonic acid. The monovalent or polyvalent cation in forming the salt structure is not particularly limited. Examples thereof include an inorganic cation and an organic cation, and specific examples thereof include alkali metal cations such as Na⁺, Li⁺, and K⁺, alkaline earth metal cations such as Mg²⁺, Ca²⁺, and Ba²⁺, and organic ammonium cations such as a trialkylammonium cation and a tetraalkylammonium cation.

In a case of a salt structure, the kind of the salt may be one kind, two or more kinds thereof may be mixed, a salt-type group and a group having a free acid structure may be mixed in a compound, and a compound having a salt structure and a compound having a free acid structure compound may be mixed.

Any compound according to the embodiment of the present invention is an electrically neutral compound. Specifically, the charge of the compound as a whole is adjusted to be 0 by a group having a charge or by a counterion in the compound. For example, in a case where FL has a structural moiety consisting of a cyanine dye, the formal charge of the nitrogen atom is +1, and a dissociable group such as a sulfo group in the cyanine dye has an ionic structure such as a sulfonate ion in a manner to be paired with this formal charge, and thus the compound according to the embodiment of the present invention, which has a structural moiety consisting of a cyanine dye, is a compound having a charge of 0 as a whole of the compound.

In addition, the compound according to the embodiment of the present invention is meant to include a compound having a partially changed structure is included within a range which does not impair the effect of the present invention. Further, it is meant that a compound, which is not specified to be substituted or unsubstituted, may have any substituent within the scope that does not impair the effect of the present invention. The same applies to a substituent (for example, a group represented by "alkyl group", "methyl group", "methyl") and a linking group (for example, a group represented by "alkylene group", "methylene group", "methylene"). Among such substituents, a preferred substituent in the present invention is a substituent selected from a substituent group T described later.

In the present invention, in a case where the number of carbon atoms of a certain group is specified, this number of carbon atoms means the number of carbon atoms of the entire group thereof unless otherwise specified in the present invention or the present specification. That is, in a case where this group has a form that further has a substituent, it means the total number of carbon atoms, to which the number of carbon atoms of this substituent is included.

In addition, in the present invention, the numerical range represented by using "to" means a range including the numerical values before and after "to" as the lower limit value and the upper limit value, respectively.

A fluorescent compound according to the embodiment of the present invention is represented by General Formula (1). Although details of the reason why the fluorescent compound according to the embodiment of the present invention has high water solubility required for fluorescence labeling, has a high molar absorption coefficient and a high fluorescence quantum yield, has an excellent fluorescence intensity (brightness), and can exhibit excellent light resistance as a compound or a fluorescently labeled biological substance are not clear, they are conceived to be as follows.

The fluorescent compound according to the embodiment of the present invention is, as represented by General Formula (1), is a compound having a specific phenyl group substituted with substituents R¹¹ to R¹⁵ in the phosphor moiety FL, where one of two adjacent groups among the substituents R¹¹ to R¹⁵ is a sulfo group (-SO₃⁻X⁺) and the other is a substituent. As a result, a steric repulsion effect, which is obtained by a substituent that protrudes with respect to the planar skeleton of the phosphor moiety FL and is located at at least one of the meta position or para position of the phenyl group, and an electron repulsion effect due to the sulfo group are obtained. Therefore, it is conceived that at least the intermolecular interaction between the phosphor moieties FL is suppressed, the self-association or aggregation of the fluorescent compound according to the embodiment of the present invention can be suppressed, the luminous efficacy (molar absorption coefficient × fluorescence quantum yield) is improved, the fluorescence intensity (brightness) can be improved, and the water solubility due to the sulfo group is obtained. Further, it is conceived that since one of two groups adjacent to each other among the substituents R¹¹ to R¹⁵ is a sulfo group, sufficient hydrophilicity can be ensured and concurrently, the oxidation potential of the phosphor moiety is deepened thereby being difficult to be oxidized, which makes it possible to exhibit excellent light resistance. That is, the fluorescent compound according to the embodiment of the present invention has high water solubility required for fluorescence labeling, has a high molar absorption coefficient and a high fluorescence quantum yield, has an excellent fluorescence intensity (brightness), and has excellent light resistance as a compound or a fluorescently labeled biological substance, while adjusting the absorption and luminescence wavelengths to a desired range in a wide wavelength range depending on the dye skeleton of the phosphor moiety FL.

Hereinafter, the fluorescent compound according to the embodiment of the present invention, which is represented by General Formula (1), will be described in detail.

### <Fluorescent compound represented by General Formula (1)>

The fluorescent compound according to the embodiment of the present invention (hereinafter, also referred to as "the compound according to the embodiment of the present invention"), which is represented by General Formula (1), is as follows.

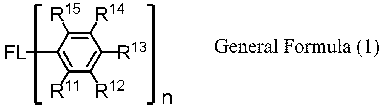

In the formula, FL indicates an n-valent phosphor moiety, and n is an integer of 1 or more.

R¹¹ to R¹⁵ represent a hydrogen atom or a substituent. However, at least one combination of the combination of R¹¹ and R¹² or the combination of R¹² and R¹³ is a combination in which one is -SO₃⁻X⁺ and the other is a substituent. X⁺ represents a hydrogen ion or a metal ion.

### (R¹¹ to R¹⁵)

R¹¹ to R¹⁵ represent a hydrogen atom or a substituent.

The substituent which can be adopted as R¹¹ to R¹⁵ is not particularly limited. Examples thereof include substituents in the substituent group T described later, and among these, preferred examples thereof include a hydroxy group, an alkoxy group, an aryloxy group, a heteroaryloxy group, a sulfo group, an alkyl group, an aryl group, a heteroaryl group, and an amino group.

Examples of the substituent which may be contained in the alkoxy group, the aryloxy group, the heteroaryloxy group, the alkyl group, the aryl group, the heteroaryl group, and the amino group, include substituents in the substituent group T which will be described later. In addition, examples thereof preferably include those having an alkylene glycol structure.

Specifically, the alkylene glycol structure is a structure represented by -(L-O)ₘ₋, where it is preferable to have the structure as a structure represented by -(L-O)ₘ₋R²¹. For example, in a case where the alkoxy group has an alkylene glycol structure, it suffices that the alkyl group moiety in the alkoxy group has an alkylene glycol structure, and it is preferable to have a structure represented by -O(L-O)ₘ₋R²¹.

The L means an alkylene group obtained by removing one hydrogen atom from an alkyl group in the substituent group T described later, where it preferably has 2 to 4 carbon atoms, more preferably has 2 or 3 carbon atoms, and still more preferably has 2 carbon atoms, and the number of carbon atoms contained in the shortest chain that links two carbon atoms which are bonding sites of the alkyl group is preferably 0 to 2, more preferably 0 or 1, and still more preferably 0.

The m means an average repetition number (simply, also referred to as a repetition number), and it is preferably 1 to 24, more preferably 1 to 12, still more preferably 1 to 10, particularly preferably 1 to 8, and most preferably 2 to 4.

The R²¹ represents an alkyl group, and it preferably has 1 to 10 carbon atoms, more preferably has 1 to 6 carbon atoms, still more preferably has 1 to 4 carbon atoms, and particularly preferably has 1 or 2 carbon atoms.

The sulfo group is a group represented by -SO₃⁻X⁺, where X⁺ represents a hydrogen ion or a metal ion. Examples of the metal ion which can be adopted as X⁺ include the cation of the alkali metal or the cation of the alkaline earth metal, which is described above.

R¹¹ to R¹⁵ are preferably a hydrogen atom, a hydroxy group, an alkoxy group, an aryloxy group, a heteroaryloxy group, or a sulfo group, more preferably a hydrogen atom, an alkoxy group, or a sulfo group, and still more preferably an alkoxy group having an alkylene glycol structure, or a hydrogen atom or a sulfo group.

However, at least one combination of the combination of R¹¹ and R¹² or the combination of R¹² and R¹³ among R¹¹ to R¹⁵ is a combination in which one is -SO₃⁻X⁺ and the other is a substituent (hereinafter, referred to as "the substituent at the ortho position"). X⁺ has the same meaning as the X⁺ described above.

The substituent at the ortho position in the above combination is preferably a hydroxy group, an alkoxy group, an aryloxy group, or a heteroaryloxy group, more preferably an alkoxy group, an aryloxy group, or a heteroaryloxy group, still more preferably an alkoxy group, and particularly preferably an alkoxy group having an alkylene glycol structure from the viewpoint that the effect of suppressing the association between dyes is excellent, the suppression of non-specific binding is excellent, the high water solubility is imparted to a compound, and the label binding property to a biological substance is excellent.

The above-described combination of -SO₃⁻X⁺ and the substituent at the ortho position is preferably a combination in which R¹² is -SO₃⁻X⁺ and R¹¹ or R¹³ is a substituent at the ortho position, or a combination in which R¹² is a substituent at the ortho position and R¹³ is -SO₃⁻X⁺, more preferably a combination in which R¹² is -SO₃⁻X⁺ and R¹¹ or R¹³ is a substituent at the ortho position, and still more preferably a combination in which R¹² is -SO₃⁻X⁺ and R¹¹ is a substituent at the ortho position.

It is noted that among R¹¹ to R¹⁵, the group that does not correspond to the above-described combination of -SO₃⁻X⁺ and the substituent at the ortho position is not particularly limited, and it is selected from the above-described hydrogen atom or substituent, which can be adopted as R¹¹ to R¹⁵.

For example, in a combination in which R¹² is -SO₃⁻X⁺ and R¹¹ is a substituent at the ortho position, it is preferable that all of R¹³ to R¹⁵ are hydrogen atoms, or R¹³ and R¹⁴ are hydrogen atoms and R¹⁵ is a substituent. In a combination in which R¹² is -SO₃⁻X⁺ and R¹³ is a substituent at the ortho position, it is preferable that all of R¹¹, R¹⁴, and R¹⁵ are hydrogen atoms, or R¹¹ is a hydrogen atom, one of R¹⁴ or R¹⁵ is a hydrogen atom, and the other thereof is a substituent. In a combination in which R¹² is a substituent at the ortho position and R¹³ is -SO₃⁻X⁺, it is preferable that R¹¹ is a hydrogen atom, one of R¹⁴ or R¹⁵ is a hydrogen atom, and the other thereof is a substituent.

The compound according to the embodiment of the present invention is preferably represented by General Formula (2).

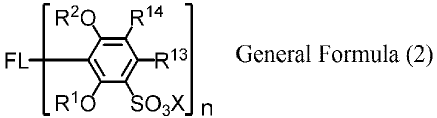

In the formula, R¹ and R² represent a hydrogen atom, an alkyl group, an aryl group, or a heteroaryl group. FL indicates an n-valent phosphor moiety, and n is an integer of 1 or more.

### (R¹ and R²)

The alkyl group which can be adopted as R¹ and R² corresponds to the alkyl group in the above-described alkoxy group which can be adopted as R¹¹ to R¹⁵, and the description of this alkyl group can be preferably applied thereto.

The aryl group which can be adopted as R¹ and R² corresponds to the aryl group in the above-described aryloxy group which can be adopted as R¹¹ to R¹⁵, and the description of this aryl group can be preferably applied thereto.

The heteroaryl group which can be adopted as R¹ and R² corresponds to the heteroaryl group in the above-described heteroaryloxy group which can be adopted as R¹¹ to R¹⁵, and the description of this heteroaryl group can be preferably applied thereto.

R¹ and R² described above are preferably an alkyl group, an aryl group, or a heteroaryl group and more preferably an alkyl group. From the viewpoint that the effect of suppressing the association between dyes is excellent, the suppression of non-specific binding is excellent, the high water solubility is imparted to a compound, and the label binding property to a biological substance is excellent, and from the viewpoint that a more excellent fluorescence quantum yield is exhibited, R¹ and R² are still more preferably an alkyl group having an alkylene glycol structure.

R¹³, R¹⁴, X⁺, and n respectively have the same meanings as R¹³, R¹⁴, X⁺, and n described above unless otherwise specified. However, R¹³ and R¹⁴ are preferably a hydrogen atom.

### (FL and n)

In General Formulae (1) and (2), FL indicates an n-valent phosphor moiety, and n is an integer of 1 or more.

The phosphor moiety which can be adopted as FL can be used without particular limitation as long as it is a structural moiety consisting of an organic compound that exhibits fluorescence.

Examples of FL include a structural moiety consisting of a dipyrromethene dye, a rhodamine dye, a xanthene dye, a pyrrolopyrrole dye, a pyrene dye, a coumarin dye, or a cyanine dye, where a structural moiety consisting of a dipyrromethene dye, a pyrrolopyrrole dye, a pyrene dye, a coumarin dye, or a cyanine dye is preferable.

As the dipyrromethene dye, the rhodamine dye, the xanthene dye, the pyrrolopyrrole dye, the pyrene dye, the coumarin dye, or the cyanine dye, which are described above, dyes that are generally known as these dyes can be used without particular limitation.

n is an integer of 1 or more, and it is preferably an integer of 1 to 8, more preferably an integer of 1 to 6, still more preferably an integer of 2 to 4, and particularly preferably an integer of 2.

From the viewpoint of general-purpose properties in bioimaging, the compound according to the embodiment of the present invention preferably has at least one carboxy group or substituent capable of being bonded to a biological substance described later, and more preferably has, in FL, at least one carboxy group or substituent capable of being bonded to a biological substance described later.

The compound according to the embodiment of the present invention can be bonded to a biological substance by the above-described carboxy group or a substituent capable of being bonded to a biological substance, whereby a targeted labeled biological substance can be obtained. It is noted that a substituent capable of being bonded to a biological substance can be easily derived from a carboxy group by a conventional method.

In the present invention, "the substituent capable of being bonded to a biological substance" includes a substituent capable of being bonded to a biological substance, which is derived from a carboxy group.

The number of carboxy groups or substituents capable of being bonded to a biological substance, which are contained in the compound according to the embodiment of the present invention, is preferably at least 1 or more in total, and it is more preferably 1 to 3, still more preferably 1 or 2, and particularly preferably 1, from the viewpoint of the quantification of the target substance to be detected.

The FL is preferably a structural moiety consisting of a dipyrromethene dye. It is noted that the dipyrromethene dye may form a complex.

The FL is more preferably a structural moiety consisting of a dipyrromethene complex dye that forms a complex with any metal atom or metalloid atom, still more preferably a structural moiety consisting of a dipyrromethene complex dye in which a dipyrromethene ligand is coordinated to a boron atom, a zinc atom, an aluminum atom, a phosphorus atom, or the like, particularly preferably a structural moiety consisting of a dipyrromethene boron complex dye, and most preferably a structural moiety consisting of a dipyrromethene boron complex dye represented by General Formula (6).

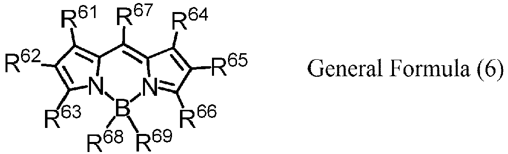

In the formula, R⁶¹ to R⁶⁷ represent a hydrogen atom or a substituent. R⁶⁸ and R⁶⁹ represent a hydrogen atom, a hydroxy group, a halogen atom, an alkoxy group, an alkyl group, an aryl group, -C≡CR^{b}, or a cyano group. R^{b} represents a hydrogen atom, an alkyl group, or an aryl group.

In a dye represented by the formula, n pieces of hydrogen atoms are removed from any one of R⁶¹ to R⁶⁹ so that the FL is n-valent.

### (i) R⁶¹ to R⁶⁷

R⁶¹ to R⁶⁷ represent a hydrogen atom or a substituent.

Examples of the substituent which can be adopted as R⁶¹ to R⁶⁷ include substituents in the substituent group T which will be described later.

Among the above, R⁶¹ and R⁶⁴ are preferably an alkyl group.

Among the above, preferred examples of R⁶² and R⁶⁵ include a hydrogen atom or an aryl group.

In a case where R⁶² or R⁶⁵ is a hydrogen atom, it is preferable that the hydrogen atom is removed to provide a bonding site of FL.

Among these, it is more preferable that R⁶² and R⁶⁵ are respectively hydrogen atoms, where at least one of these hydrogen atoms is removed to provide a bonding site of FL, and from the viewpoint of further improving the molar absorption coefficient due to an increase in integral and the like by the expansion of the highest occupied molecular orbital (HOMO) and the lowest unoccupied molecular orbital (LUMO) or the overlap thereof, it still more preferable that R⁶² and R⁶⁵ are respectively hydrogen atoms, where both of these hydrogen atoms are removed to provide a bonding site of FL.

Among the above, preferred examples of R⁶³ and R⁶⁶ include a hydrogen atom, an alkyl group, an aryl group, -CR^{a1}=CR^{a2}R^{a3}, or -C≡CR^{b}.

Examples of the substituent which may be contained in the alkyl group and aryl group described above include substituents in the substituent group T which will be described later, examples of which include a carboxy group.

R^{a1} to R^{a3} described above represent a hydrogen atom, an alkyl group, or an aryl group, and examples of the alkyl group and the aryl group include an alkyl group and an aryl group in the substituent group T which will be described later. Examples of the substituent which may be contained in this alkyl group and this aryl group include substituents in the substituent group T which will be described later, examples of which include an alkyl group, an alkoxy group, and an amino group. R^{a1} and R^{a2} are preferably a hydrogen atom, and R^{a3} is preferably a hydrogen atom or an aryl group and more preferably an aryl group.

To the R^{b}, the description of R^{b} in R⁶⁸ and R⁶⁹ can be preferably applied.

In a case where R⁶³ or R⁶⁶ is a hydrogen atom, it is preferable that the hydrogen atom is removed to provide a bonding site of FL. In addition, in a case where R^{a3} is a hydrogen atom, it is preferable that the hydrogen atom is removed to provide a bonding site of FL.

Among these, R⁶³ and R⁶⁶ are more preferably an alkyl group, an aryl group, or -CH=CR^{a3}H (however, R^{a3} is an aryl group).

Among the above, R⁶⁷ is preferably a hydrogen atom, an alkyl group, an aryl group, or a heteroaryl group, and it is more preferably an aryl group.

Examples of the substituent which may be contained in the alkyl group, aryl group, and aryl group described above include substituents in the substituent group T which will be described later, examples of which include a halogen atom (preferably a fluorine atom), an alkyl group, an alkoxy group, a carbamoyl group, a carboxy group, or a substituent capable of being bonded to a biological substance. In addition, preferred examples thereof include the group represented by -O(L-O)ₘ-R²¹ described above.
in a case where R⁶⁷ is a hydrogen atom, it is also preferable that the hydrogen atom is removed to provide a bonding site of FL.

Among these, R⁶⁷ is still more preferably a phenyl group having a substituent at the ortho position from the viewpoint that the association and aggregation between molecules can be suppressed by steric hindrance, the fluorescence quantum yield can be further improved, and the light resistance can be further improved. In the phenyl group having a substituent at the ortho position, a substituent may be or may not be provided at the meta position and the para position; however, it is preferable that a carboxy group or a substituent capable of being bonded to a biological substance is provided at the para position directly or through a linking group.

Two adjacent groups among R⁶¹ to R⁶⁷ may be bonded to each other to form a 5- or 6-membered ring. In this case, the ring to be formed may have an unsaturated bond.

For example, R⁶¹ and R⁶² may be bonded to each other to form a 5- or 6-membered ring and preferably a benzene ring, and R⁶⁴ and R⁶⁵ may be bonded to each other to form a 5- or 6-membered ring and preferably a benzene ring.

In addition, the 5- or 6-membered ring that is formed by bonding two adjacent groups among R⁶¹ to R⁶⁷ may have a substituent, and examples of the substituent which may be contained therein include substituents in the substituent group T which will be described later, examples of which include an alkoxy group and a carboxy group.

In addition, it is also preferable that a hydrogen atom on a 5- or 6-membered ring that is formed by bonding two adjacent groups among R⁶¹ to R⁶⁷ is removed to provide a bonding site of FL.

### (ii) R⁶⁸ and R⁶⁹

R⁶⁸ and R⁶⁹ represent a hydrogen atom, a hydroxy group, a halogen atom, an alkoxy group, an alkyl group, an aryl group, -C≡CR^{b}, or a cyano group. R^{b} represents a hydrogen atom, an alkyl group, or an aryl group.

Examples of the halogen atom, the alkoxy group, the alkyl group, and the aryl group, which can be adopted as R⁶⁸ or R⁶⁹, include a halogen atom, an alkoxy group, an alkyl group, and an aryl group in the substituent group T which will be described later.

Examples of the alkyl group and the aryl group which can be adopted as the R^{b} include an alkyl group and an aryl group in the substituent group T which will be described later.

Examples of the substituent which may be contained in the above-described alkoxy group, alkyl group, and aryl group, which can be adopted as R⁶⁸ or R⁶⁹, and examples of the alkyl group and the aryl group, which can be adopted as the R^{b}, include substituents in the substituent group T which will be described later, examples of which include a halogen atom (preferably a fluorine atom) and an alkyl group.

In a case where R⁶⁸ or R⁶⁹ is a hydrogen atom, it is preferable that the hydrogen atom is removed to provide a bonding site of FL. In addition, in a case where R^{b} is a hydrogen atom, it is preferable that the hydrogen atom is removed to provide a bonding site of FL.

Regarding R⁶⁸ and R⁶⁹, it is preferable that one of them is a halogen atom and the other thereof is a halogen atom, an alkyl group, a halogenated alkyl group, an aryl group, or a halogenated aryl group, it is more preferable that one of them is a halogen atom and the other thereof is a halogen atom, or a halogenated alkyl group, and from the viewpoint of further improving light resistance, it is still more preferable that one of them is a halogen atom and the other thereof is a halogenated alkyl group.

The structural moiety consisting of the dipyrromethene boron complex dye represented by General Formula (6) preferably has a carboxy group or a substituent capable of being bonded to a biological substance in at least any one of R⁶¹, ..., or R⁶⁹, more preferably has a carboxy group or a substituent capable of being bonded to a biological substance in at least any one of R⁶¹, ..., or R⁶⁷, and still more preferably has a carboxy group or a substituent capable of being bonded to a biological substance in R⁶⁷.

In addition, from the viewpoint of exhibiting sufficient hydrophilicity as the compound according to the embodiment of the present invention, in the compound according to the embodiment of the present invention which has, as FL, the structural moiety consisting of the dipyrromethene boron complex dye represented by General Formula (6), the number of hydrophilic groups per one molecule of the dipyrromethene boron complex dye represented by General Formula (6) is preferably 2 or more, more preferably 2 to 10, still more preferably 3 to 9, and particularly preferably 4 to 7.

The hydrophilic group is not particularly limited; however, examples thereof include an alkoxy group having a substituent, a carboxy group, a sulfo group, and a phosphono group, where an alkoxy group having a substituent, or a sulfo group is preferable. The alkoxy group having a substituent is preferably an alkoxy group having an alkylene glycol structure.

The hydrophilic group may be any hydrophilic group that is provided as -SO₃⁻X⁺ on the phenyl group having, as a substituent, R¹¹ to R¹⁵ defined by General Formula (1) described above, or another hydrophilic group may be provided. The number of sulfo groups per one molecule of the dye is preferably 2 or more.

In the dipyrromethene boron complex dye represented by General Formula (6), n pieces of hydrogen atoms are removed from any of R⁶¹ to R⁶⁹ so that FL is n-valent. For example, one hydrogen atom is removed from R⁶², and one hydrogen atom is removed from R⁶⁵ so that FL is divalent. In addition, two hydrogen atoms are removed from R⁶³, and two hydrogen atoms are removed from R⁶⁶ so that FL is tetravalent.

In the present invention, it is preferable that n pieces of hydrogen atoms are removed from any of R⁶² to R⁶⁹ so that FL is n-valent, it is more preferable that one hydrogen atom is removed from at least one of R⁶², ..., or R⁶⁵ so that FL is n-valent, and it still more preferable that one hydrogen atom is removed from each of R⁶² and R⁶⁵ so that FL is at least divalent.

In the compound according to the embodiment of the present invention, it is preferable that R⁶³ and R⁶⁶ described above are an alkyl group or an aryl group.

In a case where R⁶³ and R⁶⁶ are an alkyl group, the compound according to the embodiment of the present invention which has, as FL, a structural moiety consisting of a dipyrromethene boron complex dye represented by General Formula (6), can exhibit green to yellow to orange fluorescence. On the other hand, in a case where R⁶³ and R⁶⁶ are an aryl group, the compound according to the embodiment of the present invention which has, as FL, a structural moiety consisting of a dipyrromethene boron complex dye represented by General Formula (6), can exhibit orange to red fluorescence.

In addition, in the compound according to the embodiment of the present invention, it is also preferable that R⁶³ and R⁶⁶ described above are -CH=CR^{a3}H (however, R^{a3} is an aryl group), that is, the dipyrromethene boron complex dye is represented by General Formula (7). The compound according to the embodiment of the present invention which has, as FL, a structural moiety consisting of a dipyrromethene boron complex dye represented by General Formula (7), can exhibit red to deep red fluorescence. Further, in a case where an electron-donating group such as an amino group is introduced, the absorption wavelength can be further lengthened, and the fluorescence in the near-infrared wavelength range can be exhibited.

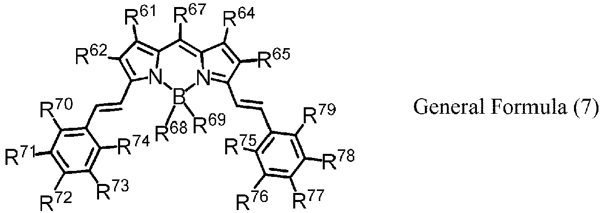

In the formula, R⁶¹, R⁶², R⁶⁴, R⁶⁵, and R⁶⁷ to R⁶⁹ respectively have the same meanings as R⁶¹, R⁶², R⁶⁴, R⁶⁵, and R⁶⁷ to R⁶⁹ in General Formula (6). R⁷⁰ to R⁷⁹ represent a hydrogen atom or a substituent.

In a dye represented by the formula, n pieces of hydrogen atoms are removed from any one of R⁶¹, R⁶², R⁶⁴, R⁶⁵, R⁶⁷ to R⁶⁹, or R⁷⁰ to R⁷⁹ so that the FL is n-valent.

R⁷⁰ to R⁷⁹ represent a hydrogen atom or a substituent.

Examples of the substituent which can be adopted as R⁷⁰ to R⁷⁹ include substituents in the substituent group T which will be described later, and preferred examples thereof include a hydrogen atom, an alkyl group, an alkoxy group, and an amino group. In addition, preferred examples thereof include the group represented by -O(L-O)ₘ-R²¹ described above.

Examples of the substituent which may be contained in the alkyl group, the alkoxy group, and the amino group, which can be adopted as R⁷⁰ to R⁷⁹, include substituents in the substituent group T which will be described later, examples of which include a halogen atom, an alkyl group, and an aryl group.

In a case where R⁷⁰ to R⁷⁹ are a hydrogen atom, it is preferable that the hydrogen atom is removed to provide a bonding site of FL. In addition, In a case where the amino group is -NH₂, it is preferable that a hydrogen atom on the nitrogen atom is removed to provide a bonding site of FL.

R⁷⁰ to R⁷⁹ are preferably a hydrogen atom.

Specific examples of the compound according to the embodiment of the present invention will be shown below; however, the present invention is not limited to these compounds. In the following specific examples, X⁺ in -SO₃⁻X⁺ and -COO⁻X⁺ has the same meaning as the above-described X⁺ and represents a hydrogen ion or a metal ion. In addition, Me represents a methyl group, and Ph represents a phenyl group.

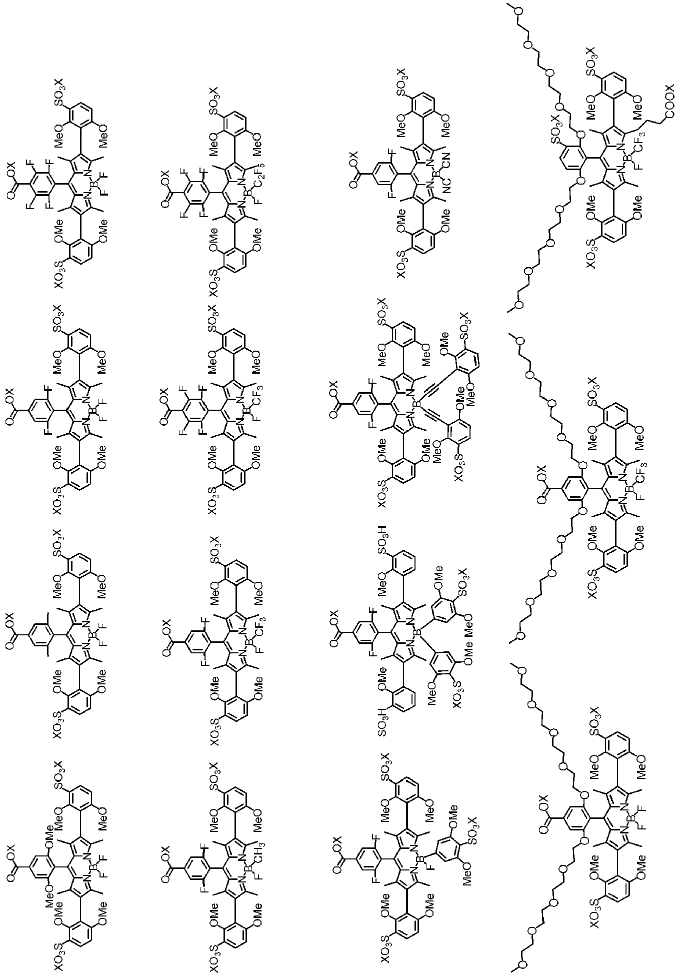

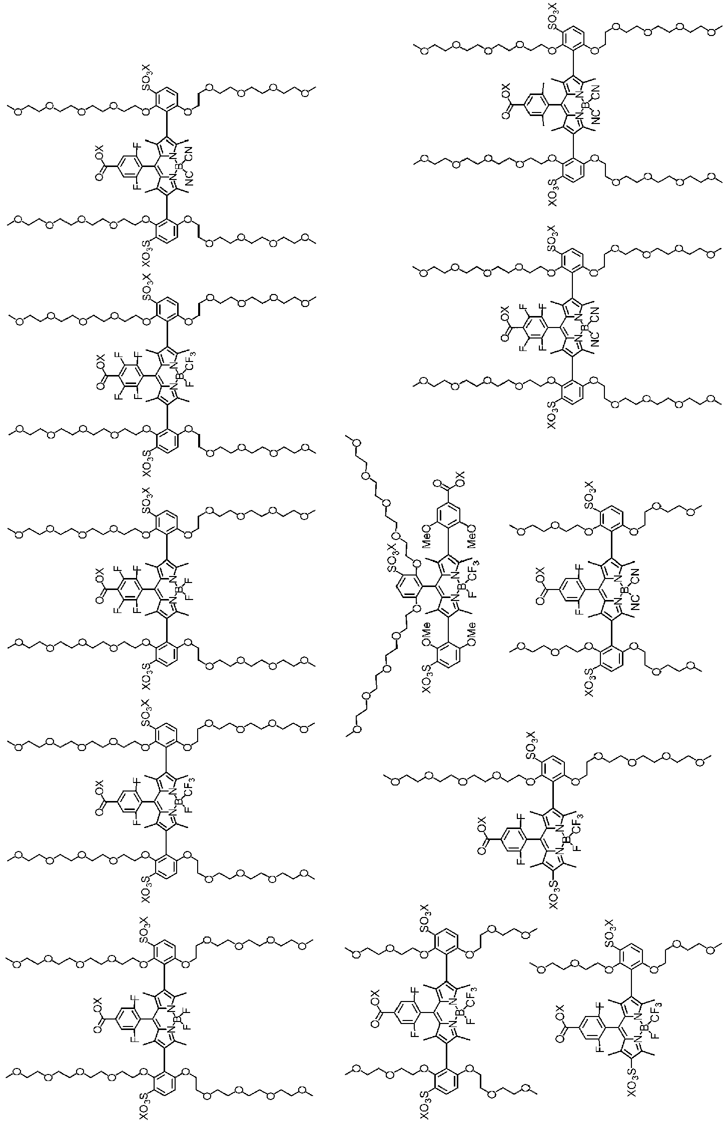

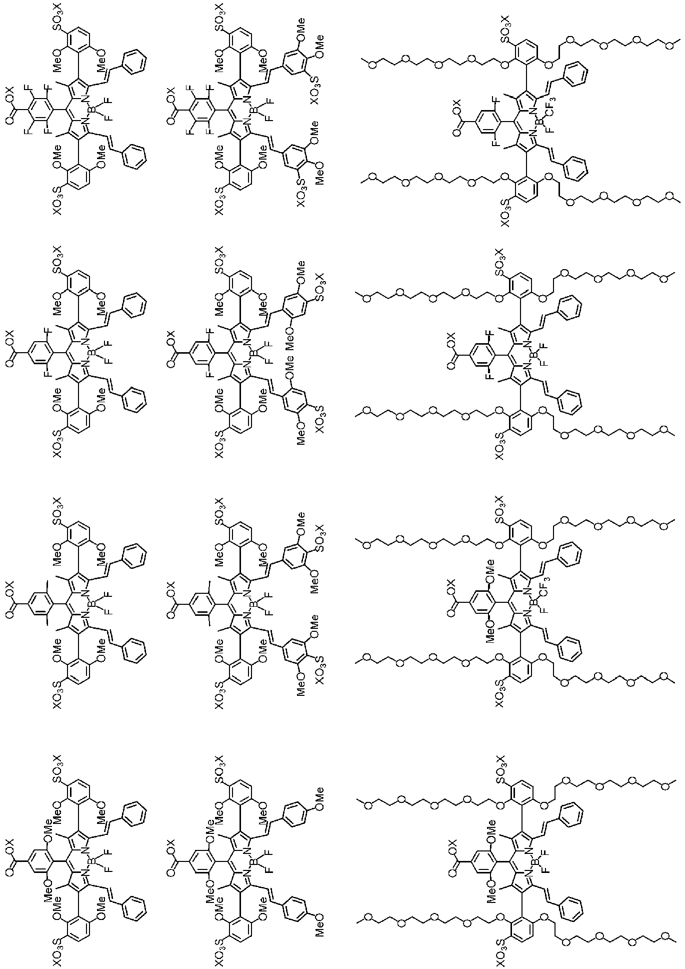

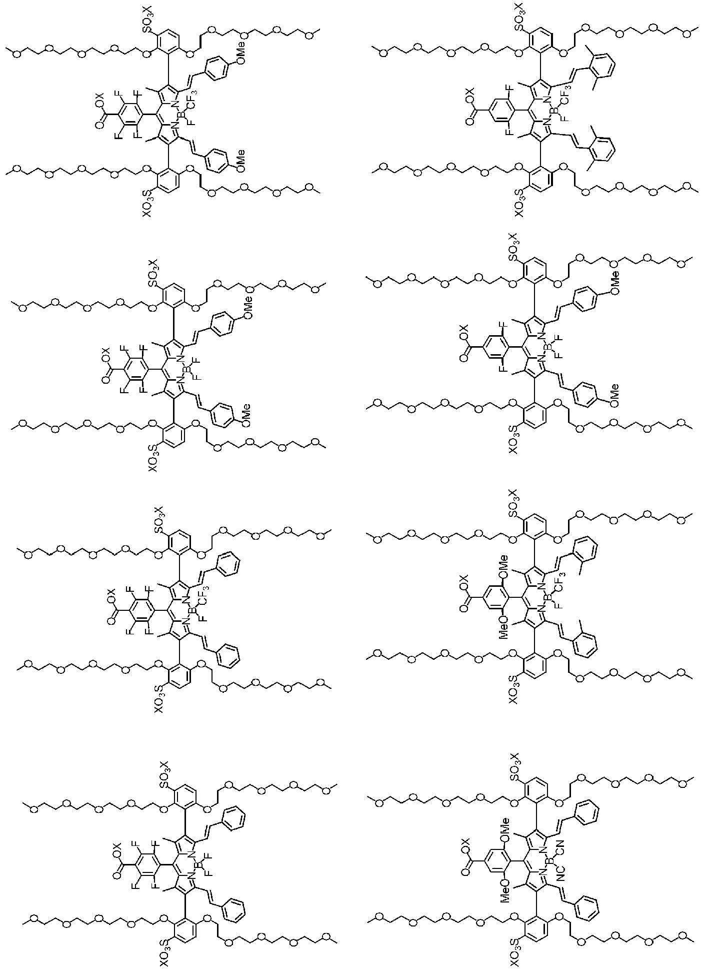

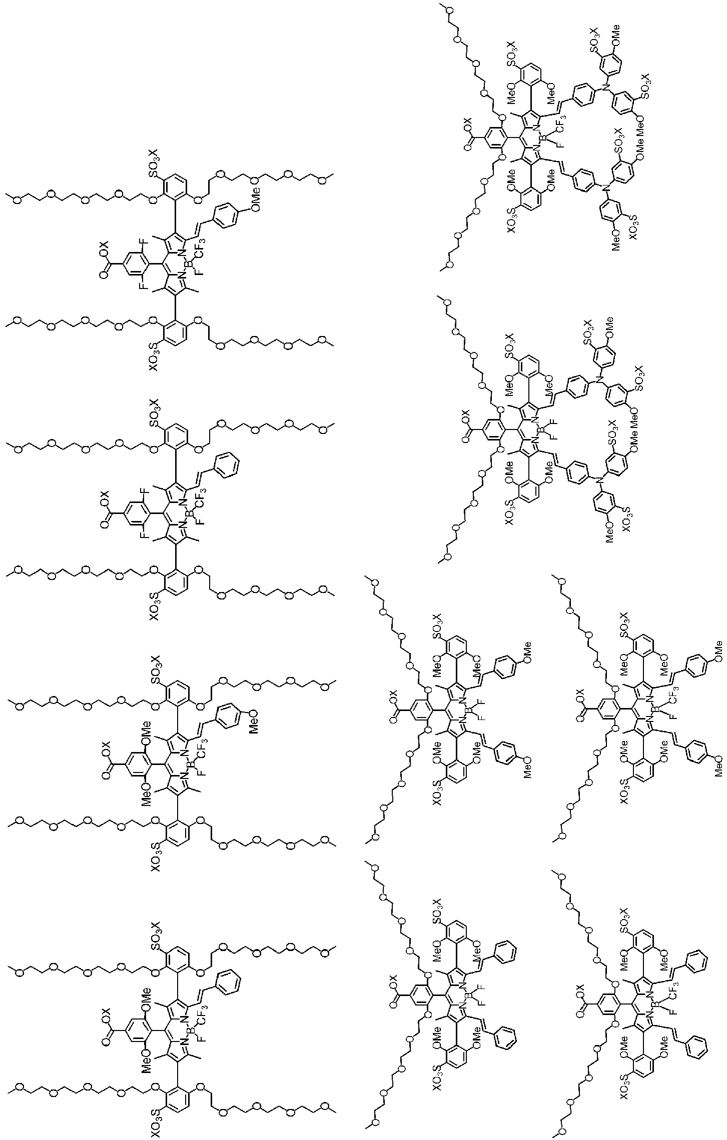

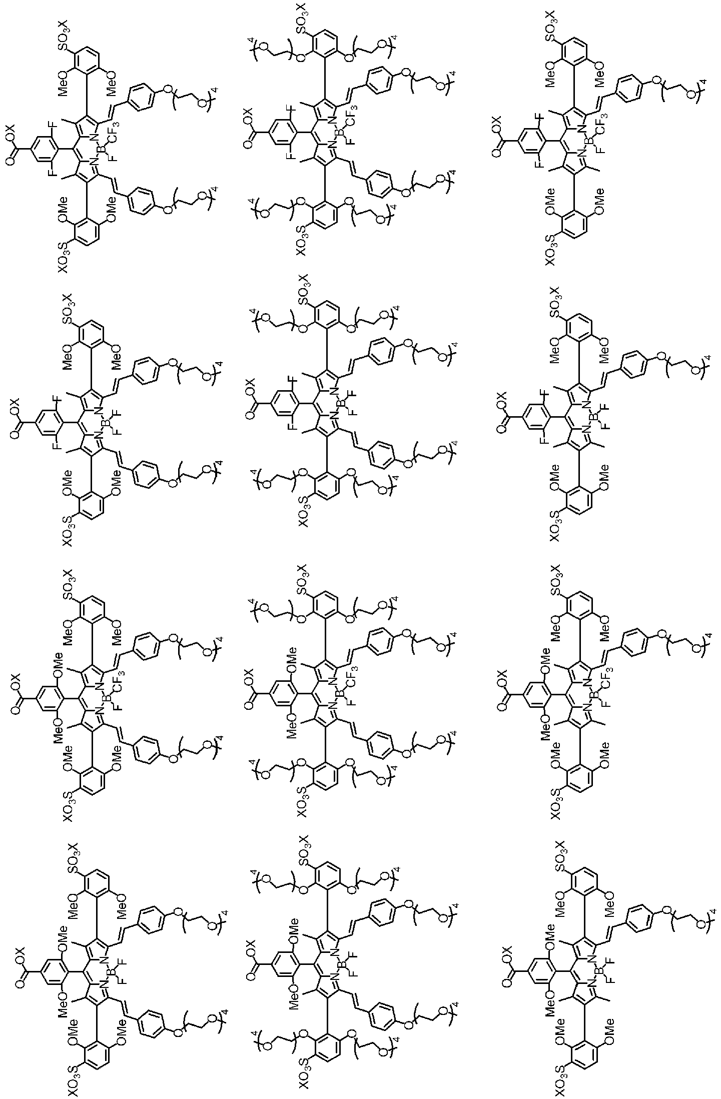

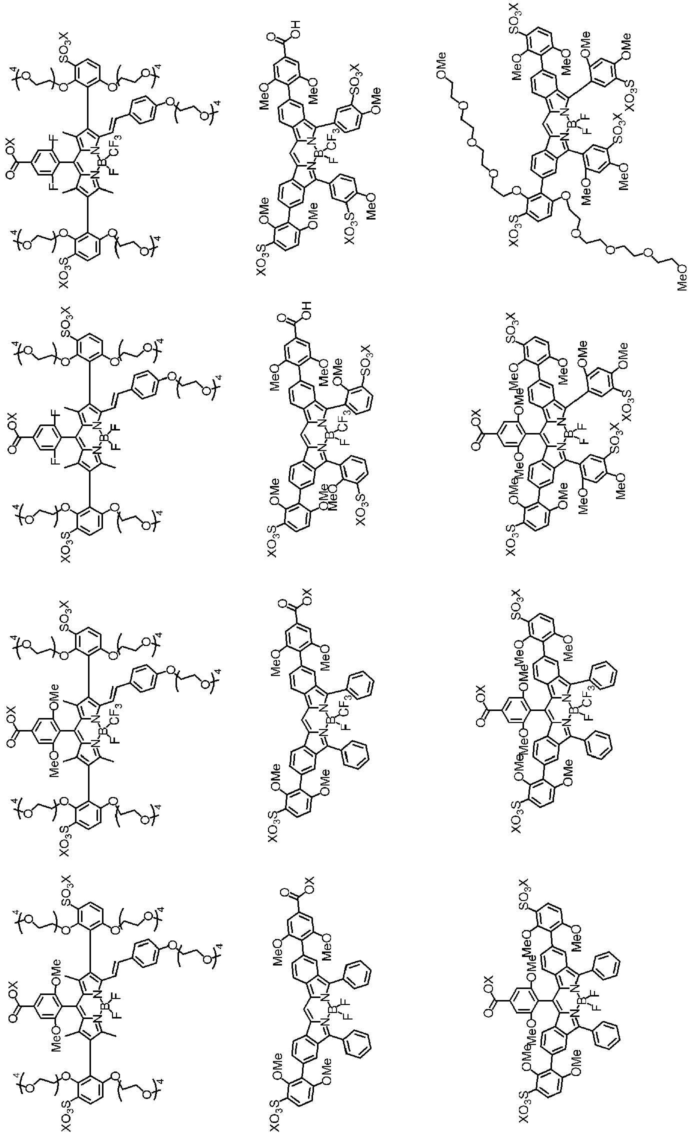

Examples of the preferred form according to the present invention include the compound represented by General Formula (2), where n is 2, FL is represented by General Formula (6), and the compound is a structural moiety consisting of a dipyrromethene boron complex dye that satisfies the following form I or II.

### (Form I)

A form in which R⁶³ and R⁶⁶ are an alkyl group or an aryl group, R⁶¹ and R⁶⁴ are an alkyl group, R⁶² and R⁶⁵ are respectively hydrogen atoms, where both of these hydrogen atoms are removed to provide a bonding site of FL, R⁶⁷ is a phenyl group that has a substituent (preferably a halogen atom or an alkoxy group) at the ortho position and has a carboxy group or a substituent capable of being bonded to a biological substance, at the para position directly or through a linking group, one of R⁶⁸ and R⁶⁹ is a halogen atom, and the other thereof is a halogen atom or a halogenated alkyl group.

### (Form II)

A form in which R⁶³ and R⁶⁶ are -CH=CR^{a3}H (however, R^{a3} is an aryl group), R⁶¹ and R⁶⁴ are an alkyl group, R⁶² and R⁶⁵ are respectively hydrogen atoms, where both of these hydrogen atoms are removed to provide a bonding site of FL, R⁶⁷ is a phenyl group that has a substituent (preferably a halogen atom or an alkoxy group) at the ortho position and has a carboxy group or a substituent capable of being bonded to a biological substance, at the para position directly or through a linking group, one of R⁶⁸ and R⁶⁹ is a halogen atom, and the other thereof is a halogen atom or a halogenated alkyl group.

In these forms, the corresponding descriptions of R⁶¹ to R⁶⁹ described above can be applied to the descriptions of R⁶¹ to R⁶⁹ in General Formula (6). In addition, the preferred descriptions pertaining to R¹, R², R¹³, R¹⁴, and X⁺ described above can be applied to R¹, R², R¹³, R¹⁴, and X⁺ in General Formula (2), and among the above, it is preferable that R¹ and R² are an alkyl group and R¹³ and R¹⁴ are a hydrogen atom.

In a case where the compound according to the embodiment of the present invention has a substituent capable of being bonded to a biological substance, the compound can be bonded to a biological substance such as a protein, a peptide, an amino acid, a nucleic acid, a sugar chain, or a lipid, by at least one substituent capable of being bonded to a biological substance, where the substituent is contained in the compound, and the compound can be used as a labeled biological substance.

The substituent capable of being bonded to a biological substance can be used without particular limitation as long as it is a group for acting (including adhering) or bonding to a biological substance, and examples thereof include the substituents described in WO2002/026891A. Among them, preferred examples thereof include an N-hydroxysuccinimide ester structure (an NHS structure), a succinimide structure, a maleimide structure, an azide group, an acetylene group, a peptide structure (a polyamino acid structure), a long-chain alkyl group (preferably having 12 to 30 carbon atoms), and a quaternary ammonium group.

Among the compounds according to the embodiment of the present invention, specific examples of the compound having at least one substituent capable of being bonded to a biological substance include, as a specific example, a form in which the carboxy group in the exemplary compound of the compound according to the embodiment of the present invention is appropriately replaced with a substituent capable of being bonded to a biological substance, which will be described later. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention can be synthesized with reference to a conventional method and a known method, except that the compound structure thereof is to be a structure defined by General Formula (1). For example, examples of the method for the synthesis of the benzene ring structure having, as a substituent, R¹¹ to R¹⁵ defined by General Formula (1) described above include the method described in the Journal of the Chemical Society of Japan (Chemistry and Industrial Chemistry), 1985, No. 1, p. 70 to 74, Acta Chemica Scandinavica, Series B: Organic Chemistry and Biochemistry, 1979, Vol. B33, p. 261-264, Journal of the Chemical Society, Perkin Transactions 2: Physical Organic Chemistry (1972-1999), 1985, No. 5, p. 659 to 667, and the like, and examples of the method for the synthesis of the dipyrromethene boron complex dye represented by General Formula (6) include the method described JP2019-172826A or WO2021/100814A. In addition, it can be synthesized in accordance with the synthesis method for each compound described in Examples which will be described later. In particular, the compound represented by General Formula (2) can be easily synthesized appropriately based on the common technical knowledge with reference to the synthesis examples described in Examples of the present application.

A compound having a substituent capable of being bonded to a biological substance can be synthesized by a known method, except that the compound structure thereof is to be the structure defined by General Formula (1). For example, Bioconjugate Techniques (Third Edition, written by Greg T. Hermanson) can be referred to.

### «Labeled biological substance»

The labeled biological substance according to the embodiment of the present invention is a substance in which the compound according to the embodiment of the present invention, is bonded to a biological substance. Since the compound according to the embodiment of the present invention has fluorescence, exhibits high hydrophilicity and an excellent fluorescence intensity, and exhibits excellent light resistance, it can be preferably used for a labeled biological substance. The bond between the compound represented by General Formula (1) and a biological substance may have a form in which the compound represented by General Formula (1) and the biological substance are directly bonded or a form in which they are linked through a linking group.

Preferred examples of the biological substance include a protein (including a peptide), an amino acid, a nucleic acid, a sugar chain, and a lipid. Preferred examples of the protein include an antibody, and preferred examples of the lipid include a phospholipid, a fatty acid, and sterol, where a phospholipid is more preferable.

Among the above biological substances, the clinically useful substance is not particularly limited; however, examples thereof include immunoglobulins such as immunoglobulin (Ig) G, IgM, IgE, IgA, and IgD; blood plasma proteins such as complement, C-reactive protein (CRP), ferritin, α₁ microglobulin, β₂ microglobulin, and antibodies thereof; tumor markers such as α-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotropin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphtheriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELM3, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, styrelidine O), and the like, and antibodies thereof. In addition, antibody fragments such as Fab'2, Fab, and Fv can also be used.

The specific form in which the compound according to the embodiment of the present invention and a biological substance interact with each other to be bonded to each other includes, for example, the forms described below.
i) A non-covalent bond (for example, hydrogen bond, ionic bond including chelate formation) or a covalent bond between a peptide in the compound according to the embodiment of the present invention and a peptide in the biological substance,
ii) a Van der Waals force between a long-chain alkyl group in the compound according to the embodiment of the present invention and a lipid bilayer, a lipid, or the like in the biological substance,
iii) an amide bond formed by reacting an N-hydroxysuccinimide ester (NHS ester) in the compound according to the embodiment of the present invention with an amino group in the biological substance,
iv) a thioether bond formed by reacting a maleimide group in the compound according to the embodiment of the present invention with a sulfanyl group (-SH) in the biological substance, and
v) a formation of a triazole ring, which is formed by a Click reaction between an azide group in the compound according to the embodiment of the present invention and an acetylene group in the biological substance, or a Click reaction between an acetylene group in the compound according to the embodiment of the present invention and an azide group in the biological substance.

In addition to the forms i) to v) described above, the bonding can be formed, for example, in the form described in Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, p. 62-83. In addition, the method described in the same document can be appropriately referred to for the preparation of the labeled biological substance according to the embodiment of the present invention.

Among the compounds according to the embodiment of the present invention, the labeled biological substance according to the embodiment of the present invention, which is obtained from a compound having a substituent capable of being bonded to a biological substance and a biological substance that is bonded to the compound by an interaction includes the compound in which a moiety other than the substituent capable of being bonded to a biological substance is replaced with a dye moiety in the compound according to the embodiment of the present invention, and a product thereof, in the description of the compound example and the product in paragraph 0038 of JP2019-172826A. However, the present invention is not limited to these compounds or the like.

### <Reagent containing labeled biological substance>

In the reagent containing the labeled biological substance according to the embodiment of the present invention, the form of the labeled biological substance according to the embodiment of the present invention, for example, a solution form dissolved in an aqueous medium such as saline and a phosphate buffer solution, and a solid form such as a fine particle powder or a lyophilized powder, is not particularly limited and can be appropriately selected depending on the purpose of use.

For example, in a case where the labeled biological substance according to the embodiment of the present invention is used as a fluorescence labeling reagent, it can be used as a reagent containing the labeled biological substance having any one of the forms described above.

### <Use application of labeled biological substance>

The labeled biological substance according to the embodiment of the present invention, which is obtained from the compound according to the embodiment of the present invention, can exhibit an excellent fluorescence intensity and stably detect fluorescence emitted from the labeled biological substance excited by light irradiation. As a result, the labeled biological substance according to the embodiment of the present invention can be applied to various techniques using the fluorescence labeling, and it can be suitably used, for example, as a fluorescence labeling reagent in a multicolor WB or dot blotting or as a reagent for in vivo fluorescence imaging.

The fluorescence detection carried out using the labeled biological substance according to the embodiment of the present invention usually includes the following processes (i) to (iii) or (iv) to (vii). The fluorescence detection including the processes (i) to (iii) corresponds to the direct method using a primary antibody fluorescently labeled with the compound according to the embodiment of the present invention, and the fluorescence detection including the processes (iv) to (vii) corresponds to the indirect method using a secondary antibody fluorescently labeled with the compound according to the embodiment of the present invention.
(i) The process of preparing each of the following (a) and (b)
   (a) A sample containing a targeted biological substance (hereinafter, also referred to as a "target biological substance")
   (b) A labeled biological substance according to the embodiment of the present invention (hereinafter, also referred to as a "labeled biological substance A according to the embodiment of the present invention") obtained by bonding the biological substance (hereinafter, also referred to as a "primary biological substance") capable of binding to the target biological substance in the above (a) to the compound according to the embodiment of the present invention
(ii) The process of preparing a conjugate (hereinafter, also referred to as a "fluorescently labeled conjugate A") in which the target biological substance in the above (a) is bonded to the primary biological substance in the labeled biological substance A according to the embodiment of the present invention in the above (b)
(iii) The process of irradiating the fluorescently labeled conjugate A with light having the range of the wavelength which is absorbed by the labeled biological substance A according to the embodiment of the present invention, and detecting the fluorescence emitted by the labeled biological substance A according to the embodiment of the present invention
(iv) The process of preparing each of the following (c) to (e)
   (c) A sample containing a target biological substance
   (d) A biological substance capable of binding to the target biological substance in the above (c) (hereinafter, also referred to as a "primary biological substance")
   (e) A labeled biological substance according to the embodiment of the present invention (hereinafter, also referred to as a "labeled biological substance B according to the embodiment of the present invention") obtained by bonding the biological substance (hereinafter, also referred to as a "secondary biological substance") capable of binding to the primary biological substance in the above (d) to the compound according to the embodiment of the present invention (hereinafter, also referred to as a "labeled biological substance B according to the embodiment of the present invention")
(v) The process of preparing a conjugate (hereinafter, also referred to as a "conjugate b") in which the target biological substance in the above (c) is bonded to the primary biological substance of the above (d)
(vi) The process of preparing a conjugate (hereinafter, also referred to as a "fluorescently labeled conjugate B2") in which the primary biological substance in the conjugate b is bonded to the secondary biological substance in the labeled biological substance B according to the embodiment of the present invention
(vii) The process of irradiating the fluorescently labeled conjugate B2 with light having the range of the wavelength which is absorbed by the labeled biological substance B according to the embodiment of the present invention, and detecting the fluorescence emitted by the labeled biological substance B according to the embodiment of the present invention

Examples of the biological substance (the primary biological substance) capable of binding to the target biological substance and the biological substance (the secondary biological substance) capable of binding to the primary biological substance include the biological substances in the labeled biological substance according to the embodiment of the present invention. The above biological substance can be appropriately selected in accordance with the target biological substance (a biological substance in the test object) or the primary biological substance, and a biological substance capable of specifically binding to the biological substance in the test object or to the primary biological substance can be selected.

Examples of the protein among the target biological substances include a protein, which is a so-called disease marker. The disease marker is not particularly limited; however, examples thereof include α-fetoprotein (AFP), protein induced by vitamin K absence or antagonist II (PIVKA-II), breast carcinoma-associated antigen (BCA) 225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, γ-seminoprotein (γ-Sm), prostate specific antigen (PSA), prostatic acid phosphatase (PAP), nerve specific enolase (NSE), Iba1, amyloid β, tau, flotillin, squamous cell carcinoma associated antigen (SCC antigen), sialyl LeX-i antigen (SLX), SPan-1, tissue polypeptide antigen (TPA), serial Tn antigen (STN), cytokeratin (CYFRA) pepsinogen (PG), C-reactive protein (CRP), serum amyloid A protein (SAA), myoglobin, creatine kinase (CK), troponin T, and ventricular muscle myosin light chain I.

The target biological substance may be a bacterium. Examples of the bacterium include a bacterium to be subjected to a cellular and microbiological test, which is not particularly limited. Specific examples thereof include Escherichia coli, Salmonella, Legionella, and bacteria causing problems in public health.

The target biological substance may be a virus. Although the virus is not particularly limited, examples of the virus include hepatitis virus antigens such as hepatitis C and B virus antigens, p24 protein antigen of HIV virus, and pp65 protein antigen of cytomegalovirus (CMV), and E6 and E7 proteins of human papillomavirus (HPV).

In the above (i) or (iv), the sample containing the target biological substance is not particularly limited and can be prepared according to a conventional method.

In addition, the labeled biological substance according to the embodiment of the present invention is not particularly limited and can be prepared by bonding a biological substance capable of binding to a target biological substance to the compound according to the embodiment of the present invention, according to a conventional method. The form of the bond and the reaction to form the bond are as described above in the labeled biological substance according to the embodiment of the present invention.

In the above (v), the target biological substance may be directly bonded to the primary biological substance or may be bonded through another biological substance which is different from the target biological substance and the primary biological substance. In addition, in the above (vi), the primary biological substance in the conjugate b may be directly bonded to the secondary biological substance in the labeled biological substance B according to the embodiment of the present invention or may be bonded through another biological substance which is different from the primary biological substance and the secondary biological substance.

The labeled biological substance according to the embodiment of the present invention can be used as a fluorescently labeled antibody in both the direct method and the indirect method but is preferably used as a fluorescently labeled antibody in the indirect method.

In the above (ii) or (v) and the above (vi), the binding of the labeled biological substance or the like according to the embodiment of the present invention to the target biological substance is not particularly limited and can be carried out according to a conventional method.

In the above (iii) or (vii), the wavelength for exciting the labeled biological substance according to the embodiment of the present invention is not particularly limited as long as the wavelength is a luminescence wavelength (wavelength light) capable of exciting the labeled biological substance according to the embodiment of the present invention. Generally, the wavelength for excitation is preferably 300 to 1,000 nm and more preferably 400 to 800 nm.

The fluorescence excitation light source used in the present invention is not particularly limited as long as it emits light having a luminescence wavelength (wavelength light) capable of exciting the labeled biological substance according to the embodiment of the present invention, and for example, various laser light sources can be used. In addition, various optical filters can be used to obtain a preferred excitation wavelength or detect only fluorescence.

Other matters in the above (i) to (vii) are not particularly limited, and conditions of a method, a reagent, a device, and the like, which are generally used in the fluorescence detection using fluorescence labeling, can be appropriately selected.

In addition, also regarding the processes other than the above (i) to (vii), conditions of a method, a reagent, a device, and the like, which are generally used, can be appropriately selected in accordance with various methods using fluorescence labeling.

For example, in the multicolor WB using the labeled biological substance according to the embodiment of the present invention, it is possible to stably detect fluorescence emitted from a target biological substance with excellent fluorescence intensity by preparing a blotted membrane according to a method generally used for a target biological substance (protein separation by electrophoresis, blotting to a membrane, and blocking of a membrane) and using the labeled biological substance according to the embodiment of the present invention as a labeled antibody (preferably, as a secondary antibody). In the dot blotting using the labeled biological substance according to the embodiment of the present invention, as in the case of the multicolor WB, it is possible to stably detect fluorescence emitted from a target biological substance with excellent fluorescence intensity by preparing a blotted nitrocellulose membrane, a blotted PVDF (polyvinylidene fluoride) membrane, or the like according to a method generally used for a target biological substance and using the labeled biological substance according to the embodiment of the present invention as a labeled antibody (preferably, as a secondary antibody).

### - Substituent group T -

In the present invention, the preferred substituents include those selected from the following substituent group T.

In addition, in the present invention, in a case of being simply described as a substituent, the substituent refers to this substituent group T, and in a case where an individual group, for example, an alkyl group is only described, a corresponding group in the substituent group T is preferably applied.

Further, in the present specification, in a case where an alkyl group is described separately from a cyclic (cyclo)alkyl group, the alkyl group is meant to include a linear alkyl group and a branched alkyl group. On the other hand, in a case where an alkyl group is not described separately from a cyclic alkyl group, and unless otherwise specified, the alkyl group is meant to include a linear alkyl group, a branched alkyl group, and a cycloalkyl group. The same applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure.

In the following description of the substituent group T, a group having a linear or branched structure and a group having a cyclic structure, such as an alkyl group and a cycloalkyl group, are sometimes described separately for clarity.

Examples of the groups included in the substituent group T include the following groups.

An alkyl group (preferably having 1 to 20 carbon atoms, more preferably having 1 to 12 carbon atoms, still more preferably having 1 to 8 carbon atoms, particularly preferably having 1 to 6 carbon atoms, and most preferably 1 to 3 carbon atoms; examples thereof include methyl, ethyl, isopropyl, t-butyl, pentyl, heptyl, 1-ethylpentyl, benzyl, 2-ethoxyethyl, 1-carboxymethyl, and trifluoromethyl), an alkenyl group (preferably having 2 to 20 carbon atoms, more preferably having 2 to 12 carbon atoms, still more preferably having 2 to 10 carbon atoms, and particularly preferably having 2 to 8 carbon atoms; examples thereof include vinyl, allyl, and oleyl), an alkynyl group (preferably having 2 to 20 carbon atoms, more preferably having 2 to 12 carbon atoms, still more preferably having 2 to 10 carbon atoms, and particularly preferably having 2 to 8 carbon atoms; examples thereof include ethynyl, butadynyl, and phenylethynyl), a cycloalkyl group (preferably having 3 to 20 carbon atoms; examples thereof include cyclopropyl, cyclopentyl, cyclohexyl, and 4-methylcyclohexyl), a cycloalkenyl group (preferably having 5 to 20 carbon atoms; examples thereof include cyclopentenyl and cyclohexenyl), an aryl group (it may be a monocyclic group or may be a fused ring group (preferably, a fused ring group having 2 to 6 rings; it preferably has 6 to 26 carbon atoms, more preferably having 6 to 18 carbon atoms, and still more preferably having 6 to 12 carbon atoms; and examples thereof include phenyl, 1-naphthyl, 4-methoxyphenyl, 2-chlorophenyl, 3-methylphenyl, and 3-methylphenyl), a heterocyclic group (preferably having 2 to 20 carbon atoms; it has at least one oxygen atom, sulfur atom, or nitrogen atom, as a ring-constituting atom, it may be a monocyclic group or may be a fused ring group (preferably, a fused ring group having 2 to 6 rings, in a case of a monocyclic group, the monocyclic group is preferably a 5-membered to 7-membered ring and more preferably a 5- or 6-membered ring, where the heterocyclic group includes an aromatic heterocyclic group (a heteroaryl group) and an aliphatic heterocyclic group (an aliphatic heterocyclic group), and examples thereof include 2-pyridyl, 4-pyridyl, 2-imidazolyl, 2-benzimidazolyl, 2-thiazolyl, and 2-oxazolyl), an alkoxy group (preferably having 1 to 20 carbon atoms and more preferably having 1 to 12 carbon atoms; examples thereof include methoxy, ethoxy, isopropyloxy, and benzyloxy), an alkynyloxy group (preferably having 2 to 20 carbon atoms and more preferably having 1 to 12 carbon atoms; examples thereof include vinyloxy and allyloxy), an alkynyloxy group (preferably having 2 to 20 carbon atoms and more preferably having 1 to 12 carbon atoms; examples thereof include 2-propynyloxy and 4-butynyloxy), a cycloalkyloxy group (preferably having 3 to 20 carbon atoms; examples thereof include cyclopropyloxy, cyclopentyloxy, cyclohexyloxy, and 4-methylcyclohexyloxy), an aryloxy group (preferably having 6 to 26 carbon atoms and more preferably having 6 to 14 carbon atoms; examples thereof include phenoxy, 1-naphthyloxy, 3-methylphenoxy, and 4-methoxyphenoxy), a heterocyclic oxy group (for the heterocyclic group moiety in the heterocyclic oxy group, the description of the heterocyclic group described above is preferably applied); examples thereof include imidazolyloxy, benzimidazolyloxy, thiazolyloxy, benzothiazolyloxy, triazinyloxy, and prinyloxy), a polyalkyleneoxy group (preferably having 2 to 40 carbon atoms and more preferably having 2 to 20 carbon atoms),
an alkoxycarbonyl group (preferably having 2 to 20 carbon atoms; examples thereof include ethoxycarbonyl and 2-ethylhexyloxycarbonyl), a cycloalkoxycarbonyl group (preferably having 4 to 20 carbon atoms; examples thereof include cyclopropyloxycarbonyl, cyclopentyloxycarbonyl, and cyclohexyloxycarbonyl), an aryloxycarbonyl group (preferably having 6 to 20 carbon atoms; examples thereof include phenyloxycarbonyl and naphthyloxycarbonyl), an amino group (preferably having 0 to 20 carbon atoms; it includes an alkylamino group, an alkynylamino group, an alkylamino group, a cycloalkylamino group, a cycloalkenylamino group, an arylamino group, and a heterocyclic amino group, where each of the above groups that substitute an unsubstituted amino group has the same meaning as the corresponding group in the substituent group T; examples thereof include amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, and triazinylamino), a sulfamoyl group (preferably having 0 to 20 carbon atoms and preferably being an alkyl, cycloalkyl, or aryl sulfamoyl group; examples thereof include N,N,-dimethylsulfamoyl, N-cyclohexylsulfamoyl, and N-phenylsulfamoyl), an acyl group (preferably having 1 to 20 carbon atoms; examples thereof include acetyl, cyclohexylcarbonyl, and benzoyl), an acyloxy group (preferably having 1 to 20 carbon atoms; examples thereof include acetyloxy, cyclohexylcarbonyloxy, and benzoyloxy), a carbamoyl group (preferably having 1 to 20 carbon atoms and preferably being an alkyl, cycloalkyl, or aryl carbamoyl group; examples thereof include N,N,-dimethylcarbamoyl, N-cyclohexylcarbamoyl, and N-phenylcarbamoyl),
an acylamino group (preferably an acylamino group having 1 to 20 carbon atoms; examples thereof include acetylamino, cyclohexylcarbonylamino, and benzoylamino), an amide group (an aminocarbonyl group), a sulfonamide group (preferably having 0 to 20 carbon atoms, and preferably being an alkyl, cycloalkyl, or aryl sulfonamide group; examples thereof include methanesulfonamide, benzenesulfonamide, N-methylmethanesulfonamide, N-cyclohexylsulfonamide, and N-ethylbenzenesulfonamide), an alkylthio group (preferably having 1 to 20 carbon atoms; examples thereof include methylthio, ethylthio, isopropylthio, and benzylthio), a cycloalkylthio group (preferably having 3 to 20 carbon atoms; examples thereof include cyclopropylthio, cyclopentylthio, cyclohexylthio, and 4-methylcyclohexylthio), an arylthio group (preferably having 6 to 26 carbon atoms; examples thereof include phenylthio, 1-naphthylthio, 3-methylphenylthio, and 4-methoxyphenylthio), an alkyl-, cycloalkyl-, or arylsulfonyl group (preferably having 1 to 20 carbon atoms; examples thereof include methylsulfonyl, ethylsulfonyl, cyclohexylsulfonyl, and benzenesulfonyl),
a silyl group (preferably having 1 to 20 carbon atoms and preferably being a silyl group substituted with an alkyl, aryl, alkoxy, or aryloxy; examples thereof include triethylsilyl, triphenylsilyl, diethylbenzylsilyl, and dimethylphenylsilyl), a silyloxy group (preferably having 1 to 20 carbon atoms and preferably being a silyloxy group substituted with an alkyl, aryl, alkoxy, or aryloxy; examples thereof include triethylsilyloxy, triphenylsilyloxy, diethylbenzylsilyloxy, and dimethylphenylsilyloxy), a hydroxy group, a cyano group, a nitro group, a halogen atom (examples thereof include a fluorine atom, a chlorine atom, a bromine atom, and an iodine atom, where a fluorine atom is preferable), a carboxy group, a sulfo group, a phosphonyl group, a phosphoryl group, a borate group, a sulfanyl group (-SH), an amino acid residue, and a polyamino acid residue. In addition, the substituent selected from the substituent group T is more preferably an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a cyano group or a halogen atom, and particularly preferably an alkyl group, an alkenyl group, a heterocyclic group, an alkoxy group, an alkoxycarbonyl group, an amino group, an acylamino group, or a cyano group.

The substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be linear or branched, and the alkyl group, the alkenyl group, or the like may be substituted or unsubstituted. In addition, in a case where an aryl group, a heterocyclic group, or the like is contained, it may be a monocyclic ring or a fused ring and may be substituted or unsubstituted.

### Examples

Hereinafter, the present invention will be described in more detail based on Examples, but the present invention is not limited thereto. It is noted that room temperature means 25°C.

Compounds (1-1) to (1-5) and (2-1) and (2-2) and comparative compounds (1) and (2), which are used in Examples, are shown below. Me indicates a methyl group.

It is noted that in the compounds of Examples, the sulfo group may include a salt structure (for example, a metal salt such as a potassium salt or a sodium salt, or a triethylamine (TEA) salt or an N,N-diisopropylethylamine (DIPEA) salt as a partial structure), even unless otherwise specified.

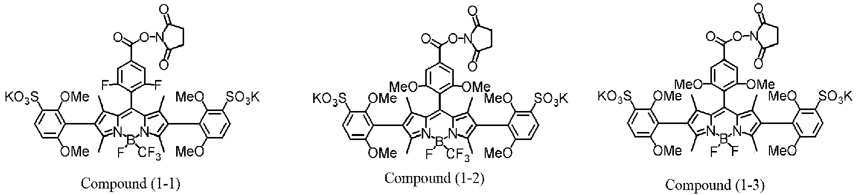

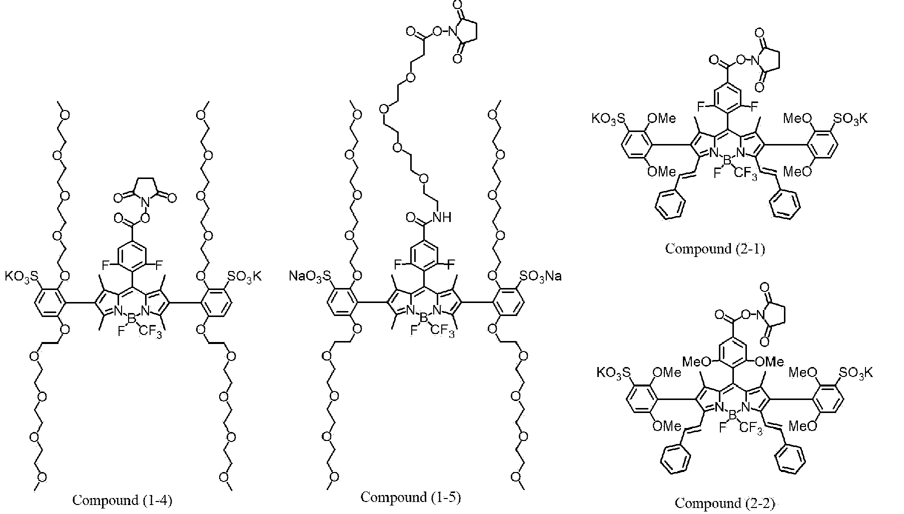

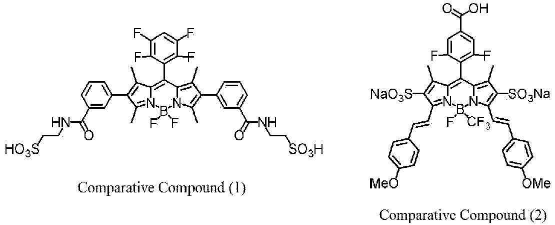

The comparative compound (1) is the compound (4) described in Examples of JP2019-172826A, and it was synthesized based on the method described in JP2019-172826A. The comparative compound (2) is the compound described secondly from the top of the left end in [Chemical 72] of WO2021/100814A, and it was synthesized according to the synthesis examples of the compounds described later with reference to the method described in WO2021/100814A. In addition, an antibody labeled with the comparative compound (2) was synthesized in the same manner except that a compound (1-F) was changed to the comparative compound (2) in the synthesis method for an antibody labeled with a compound (1-1) described later.

The method of synthesizing each compound and a labeled antibody will be described in detail below; however, the starting materials, the dye intermediates, and the synthetic routes are not limited thereto.

Unless otherwise specified, SNAP Ultra C18 (product name, manufactured by Biotage, LLC) or Sfar C18 (product name, manufactured by Biotage, LLC) was used as the carrier in the reverse phase column chromatography, and Hi-Flash Column (product name, manufactured by Yamazen Corporation) was used as a carrier in the normal phase column chromatography.

The mixing ratio in the eluent used in the reverse phase column chromatography or the normal phase column chromatography is in terms of the volume ratio. For example, "acetonitrile:water = from 0:100 to 20:80" means that the eluent of "acetonitrile:water = 0:100" was changed to an eluent of "acetonitrile:water = 20:80".

For the preparative high performance liquid chromatography (HPLC), 2767 (product name, manufactured by Waters Corporation) was used.

The MS spectrum was measured by ACQUITY SQD LC/MS System [product name, manufactured by Waters Corporation, ionization method: electrospray Ionization (ESI)] or LCMS-2010EV [product name, manufactured by Shimadzu Corporation, ionization method: an ionization method simultaneously carrying out ESI and atomospheric pressure chemical ionization (APCI)].

### [Synthesis Example 1: Synthesis of compound (1-1) and antibody labeled with compound (1-1)]

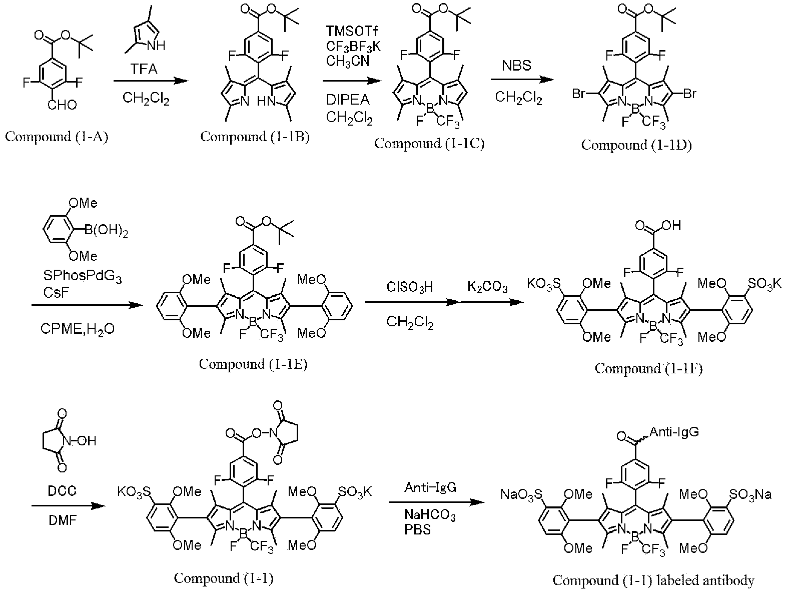

220 mg of a compound (1-1A), that is, tert-butyl 3,5-difluoro-4-formylbenzoate (manufactured by Combi-Blocks Inc.), 10 ml of dichloromethane, and 175 mg of 2,4-dimethylpyrrole were placed in a 100 ml three-neck flask, and stirring was carried out under a nitrogen stream. The reaction solution was subjected to ice-cooling in an ice bath, 1 µl of trifluoroacetic acid (TFA) was added dropwise thereto, and stirring was carried out for 2 hours. Then, 209 mg of 2,3-dichloro-5,6-dicyanobenzoquinone was added thereto, and stirring was carried out for 30 minutes. After adding 1 µl of triethylamine, dichloromethane was concentrated under reduced pressure, and purification was carried out by silica gel column chromatography using ethyl acetate/hexane as an eluent to obtain a compound (1-1B).

170 mg of the compound (1-1B), 3 ml of dichloromethane (ultra-dehydrated), and 0.86 ml of N-ethyldiisopropylamine (DIPEA) were placed in a 100 ml three-neck flask, and stirring was carried out for 10 minutes or longer. A mixed solution of 290 mg of CF₃BF₃K / 732 mg of trimethylsilyltrifluoromethanesulfonate (TMSOTF) / 3.3 ml of acetonitrile (ultra-dehydrated), which had been separately prepared, was added dropwise thereto, and stirring was carried out under nitrogen for 1 hour. Then, distilled water was added thereto, a liquid separation operation was carried out to extract the organic layer, followed by concentration under reduced pressure, and then purification was carried out by silica gel column chromatography using ethyl acetate/hexane as an eluent to obtain a compound (1-1C).

170 mg of the compound (1-1C) and 1 ml of dichloromethane were placed in a 100 ml three-neck flask, mixed and stirred, and cooled to 0°C to 5°C, and then 41 mg of N-bromosuccinimide (NBS) was added thereto, and stirring was carried out for 15 minutes. Purification was carried out by silica gel column chromatography using dichloromethane/hexane as an eluent to obtain a compound (1-1D).

35 mg of the compound (1-1D), 66 mg of 2,6-dimethoxyphenyl boronic acid, 68 mg of cesium fluoride, 0.1 ml of distilled water, and 6 ml of cyclopentyl methyl ether (CPME) were placed in a 100 ml three-neck flask, and degassing and nitrogen substitution were carried out while carrying out stirring. Then, 7 mg of SPhosPdG3 (product name, manufactured by Sigma-Aldrich Co., LLC, a Pd catalyst for cross-coupling) was added thereto, and stirring was carried out at 100°C. During the reaction, SPhosPdG3 and distilled water were appropriately added thereto, and heating and stirring were carried out. After returning the reaction solution to room temperature, distilled water was added thereto, a liquid separation operation was carried out to extract the organic layer, followed by concentration under reduced pressure, and then purification was carried out by silica gel column chromatography using ethyl acetate/hexane as an eluent to obtain a compound (1-1E).

9 mg of the compound (1-1E), 0.9 ml of dichloromethane (ultra-dehydrated), and acetonitrile (0.3 ml, ultra-dehydrated) were placed in a 50 ml eggplant flask, and stirring was carried out under nitrogen. 11 µl of chlorosulfonic acid was added under ice-cooling, and stirring was carried out for 30 minutes. 72 mg of potassium carbonate was added thereto, and then 2 ml of distilled water was added dropwise thereto, followed by stirring. The reaction solution was purified using Sfar C18 (product name, manufactured by Biotage, LLC) and using acetonitrile/water as an eluent to obtain 4.5 mg of a compound (1-1F). The identification of the obtained compound was carried out by ESI-MS. [M - 2K + 3H]⁺ = 887, [M - 2K]²⁻/2 = 442

4 mg of the compound (1-1F), 0.35 ml of N,N-dimethylformamide (DMF), and 3 mg of N-hydroxysuccinimide were placed in a 10 ml test tube, and while stirring the resultant mixture, 10 mg of N,N-dicyclohexylcarbodiimide was added thereto, and stirring was at room temperature for 1.5 hours. The reaction solution was purified using Sfar C18 (product name, manufactured by Biotage, LLC) and using acetonitrile/water as an eluent, and the solvent was distilled off by lyophilization to obtain 1 mg of a compound (1-1).

40 µL of a carbonate pH standard solution (pH = 10.01) (FUJIFILM Wako Pure Chemical Corporation) and 3.2 µL of a dimethyl sulfoxide (DMSO) solution having a concentration of the compound (1-1) of 20 mM were added to 400 µL of an anti-rabbit IgG antibody [Anti-IgG, host: goat, 2.4 mg/mL, catalog number: 111-005-003, manufactured by Jackson ImmunoResearch Inc.], and the resultant mixture was stirred and allowed to stand at room temperature for 1 hour. Subsequently, the reaction solution was directly applied onto a Sephadex G-25 column [catalog number: 17085101, manufactured by GE Healthcare] and purified with phosphate buffered saline [PBS, pH = 7.4, manufactured by FUJIFILM Wako Pure Chemical Corporation] to obtain an antibody labeled with the compound (1-1).

### [Synthesis Example 2: Synthesis of compound (1-2) and antibody labeled with compound (1-2)]

A compound (1-2) was synthesized in the same manner, except that in the synthesis of the compound (1-1), the compound (1-1A) was replaced with tert-butyl 3,5-dimethoxy-4-formylbenzoate (synthesized according to the synthesis method for the compound (7-C) described in WO2019/230963A). [M - Su (succinimide, C₄H₄NO₂, the same applies hereinafter) -2K + 4H]⁺ = 911, [M - Su (succinimide) - 2K + H]²⁻/2 = 454

An antibody labeled with the compound (1-2) was synthesized in the same manner, except that in the synthesis of the antibody labeled with the compound (1-1), the compound (1-1) was changed to the compound (1-2).

### [Synthesis Example 3: synthesis of compound (1-3)]

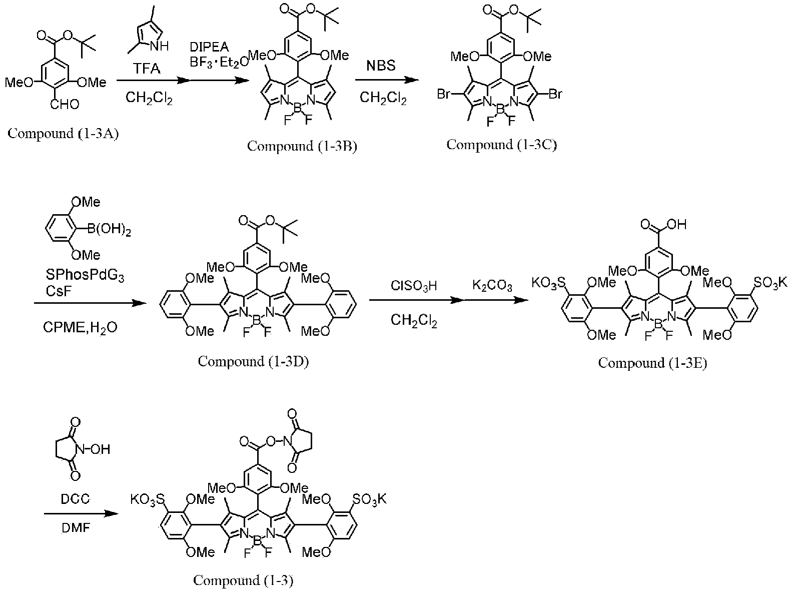

1 g of a compound (1-3A), that is, tert-butyl 3,5-dimethoxy-4-formylbenzoate (synthesized according to the synthesis method for the compound (7-C) described in WO2019/230963A), 100 ml of dichloromethane, and 686 mg of 2,4-dimethylpyrrole were placed in 100 ml three-neck flask, and stirring was carried out under a nitrogen stream. The reaction solution was subjected to ice-cooling in an ice bath, 19 µl of trifluoroacetic acid (TFA) was added dropwise thereto, and stirring was carried out for 2 hours. Then, 844 mg of 2,3-dichloro-5,6-dicyanobenzoquinone was added thereto, and stirring was carried out for 30 minutes. After adding 3.46 ml of N-ethyldiisopropylamine (DIPEA), 4.1 ml of boron trifluoride diethyl ether complex was slowly added dropwise thereto, and stirring was carried out for 1 hour. While stirring a solution, which had been obtained by adding ice to 100 ml of saturated aqueous sodium bicarbonate so that the volume was 200 ml, the reaction solution was gradually added thereto, and stirring was carried out for 10 minutes. Dichloromethane was concentrated under reduced pressure, and purification was carried out by silica gel column chromatography using ethyl acetate/hexane as an eluent to obtain a compound (1-3B).

A compound (1-3) was obtained in the same manner as in the synthesis of the compound (1-1), except that in the synthesis of the compound (1-1), the compound (1-3B) was used instead of the compound (1-1C). [M - Su (succinimide) - 2K + 4H]⁺ = 861, [M - Su (succinimide) - 2K + H]²⁻/2 = 429

### [Synthesis Example 4: Synthesis of Compound (1-4)]

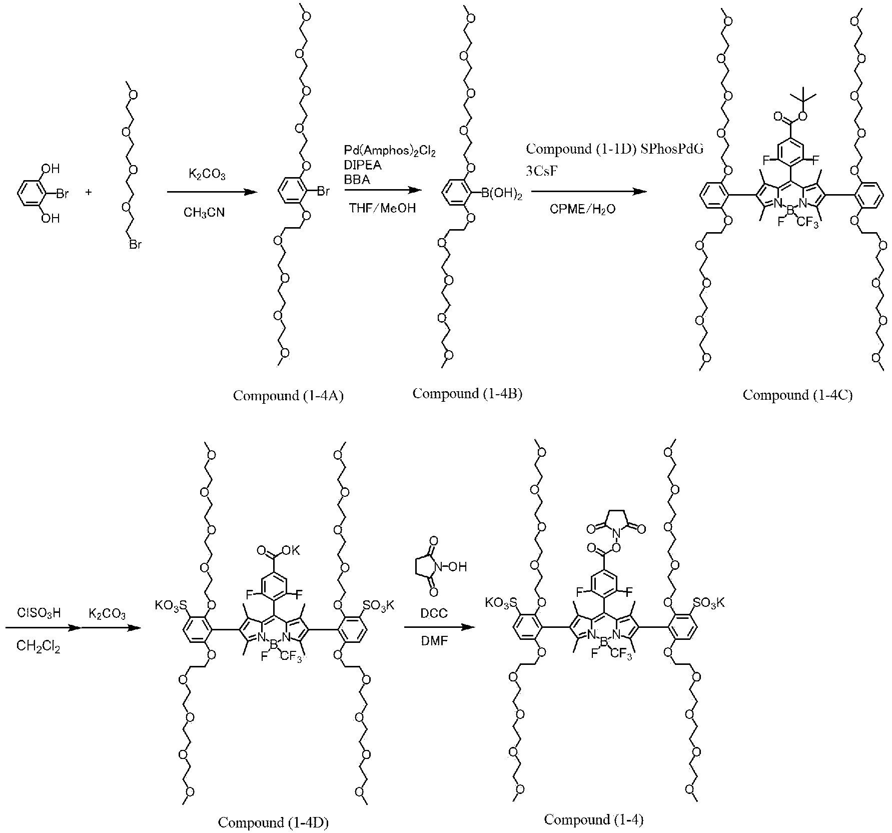

2 g of 2-bromoresorcinol, 20 ml of acetonitrile, 6.51 g of triethylene glycol-2-bromoethyl ether, and 2.87 g of potassium carbonate were placed in a 100 ml three-neck flask, and the resultant mixture was stirred at 70°C for 11 hours in a nitrogen stream to carry out a reaction. Thereafter, a liquid separation operation was carried out using ethyl acetate and distilled water, and a liquid separation operation was carried out to extract the organic layer, followed by concentration under reduced pressure, and then purification was carried out by silica gel column chromatography using ethyl acetate as an eluent to obtain 2.0 g of a compound (1-4A).

1.8 g of the compound (1-4A), 0.45 g of tetrahydroxydiboron (BBA), 40 ml of tetrahydrofuran (THF), 20 ml of methanol, and 1.7 ml of N-ethyldiisopropylamine (DIPEA) were placed in a 200 ml three-neck flask, degassing was carried out under reduced pressure while carrying out stirring, and nitrogen substitution was carried out. Subsequently, 23 mg of bis(di-tert-butyl(4-dimethylaminophenyl)phosphine)dichloropalladium (II) (Pd(Amphos)₂Cl₂) was added thereto, and heating and stirring were carried out at 58°C for 3 hours. After completion of the reaction, the reaction solution was allowed to stand overnight at room temperature, and then the solvent was distilled off under reduced pressure with an evaporator. Purification was carried out by silica gel column chromatography using ethyl acetate/methanol as an eluent to obtain 0.8 g of a compound (1-4B).

A compound (1-4) was synthesized in the same manner as in the synthesis of the compound (1-1), except that in the synthesis of the compound (1-1), the compound (1-4B) was used instead of 2,6-dimethoxyphenyl boronic acid. [M - Su (succinimide) - 2K + 4H]⁺ = 1,591.6, [M - Su (succinimide) - 2K + 2H]⁻ = 1,589.6

### [Synthesis Example 5: Synthesis of Compound (1-5)]

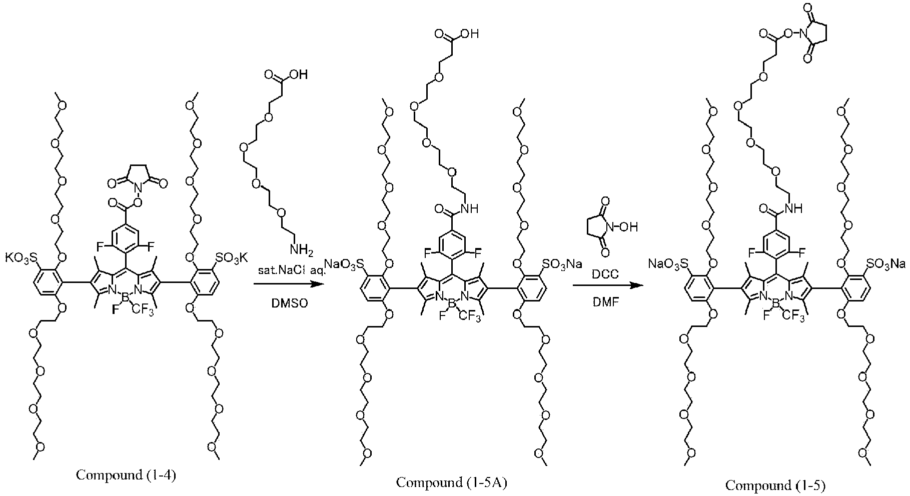

10 mg of the compound (1-4), 10 mg of 1-amino-3,6,9,12-tetraoxapentadecane-15-ic acid, 0.3 ml of DMF, and 6 µl of triethylamine were placed in a 50 ml three-neck flask, and stirring was carried out at room temperature for 10 minutes. Saturated saline was added thereto, the reaction solution was purified using Sfar C18 (product name, manufactured by Biotage, LLC) and using acetonitrile/water as an eluent, and the solvent was distilled off by lyophilization to obtain 8 mg of a compound (1-5A).

A compound (1-5) was synthesized in the same manner as in the synthesis of the compound (1-4), except that in the synthesis of the compound (1-4), the compound (1-5 A) was used instead of the compound (1-4D). [M -Su (succinimide) - 2Na + 4H]⁺ = 1,838.7, [M - Su (succinimide) - 2Na + H]²⁻/2 = 917.8

An antibody labeled with the compound (1-5) was synthesized in the same manner, except that in the synthesis of the antibody labeled with the compound (1-1), the compound (1-1) was changed to the compound (1-5).

### [Synthesis Example 6: Synthesis of compound (2-1) and antibody labeled with compound (2-1)]

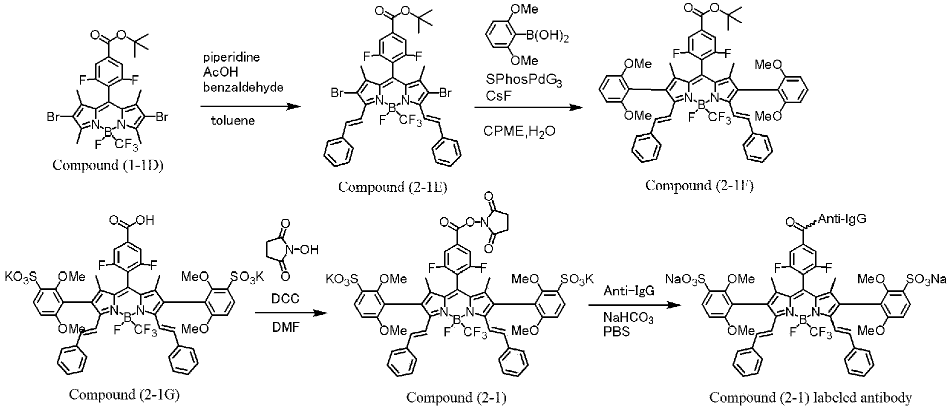

25 mg of the compound (1-1D), 5 ml of toluene (ultra-dehydrated), 30 µl of benzaldehyde, 22 µl of piperidine, and 30 µl of acetic acid (AcOH) were placed in a 100 ml three-neck flask, and heating and stirring were carried out at 90°C to 100°C under nitrogen. Distilled water was added thereto, a liquid separation operation was carried out to extract the organic layer, followed by concentration under reduced pressure, and then purification was carried out by silica gel column chromatography using ethyl acetate/hexane as an eluent to obtain a compound (2-1E).

A compound (2-1) was synthesized in the same manner as in the synthesis of the compound (1-1), except that in the synthesis of the compound (1-1), the compound (2-1E) was used instead of the compound (1-1D). [M - Su (succinimide) - 2K + 4H]⁺ = 1,063

An antibody labeled with the compound (2-1) was synthesized in the same manner, except that in the synthesis of the antibody labeled with the compound (1-1), the compound (1-1) was changed to the compound (2-1).

### [Synthesis Example 7: Synthesis of compound (2-2) and antibody labeled with compound (2-2)]

A compound (2-2) was synthesized in the same manner, except that in the synthesis of the compound (2-1), a compound obtained by replacing the compound (1-1A) in the synthesis of the compound (1-1D) with tert-butyl 3,5-dimethoxy-4-formylbenzoate (synthesized according to the synthesis method for the compound (7-C) described in WO2019/230963A) was used instead of the compound (1-1D). [M - Su (succinimide) - 2K + 4H]⁺ = 1,087

An antibody labeled with the compound (2-2) was synthesized in the same manner, except that in the synthesis of the antibody labeled with the compound (1-1), the compound (1-1) was changed to the compound (2-2).

### <Example 1> Evaluation of molar absorption coefficient and fluorescence quantum yield of compound or labeled antibody

The following characteristics were evaluated for each of the compounds, and the obtained results are shown in Table 1.

It is noted that all of the compounds (1-1) to (1-5) and (2-1) and (2-2) were dissolved in an aqueous solution to have a sample concentration of 1 mg/ml, and the high water solubility required from the viewpoint of practicality as a fluorescent compound was exhibited.

### [1] Evaluation of molar absorption coefficient ε of compound

A molar absorption coefficient ε (L/mol · cm) of a phosphate buffered saline (PBS, pH 7.4) solution of the above compound at the maximal absorption wavelength was measured using a spectrophotometer UV-3600 (product name) manufactured by Shimadzu Corporation, and evaluation was carried out based on the following evaluation standards.

Regarding the molar absorption coefficient, a high evaluation rank is highly preferable since the molar absorption coefficient is excellent, and in the present test, it is preferable that the molar absorption coefficient satisfies the evaluation rank "B" or higher (S to B) from the viewpoint of practicality as the fluorescent compound.

### - Evaluation standard for molar absorption coefficient ε -

S: 80,000 or more
A: 70,000 or more and less than 80,000
B: 60,000 or more and less than 70,000
C: 40,000 or more and less than 60,000
D: less than 40,000
In the above evaluation ranks, the unit of ε is Lmol⁻¹ cm⁻¹.

### [2] Evaluation of fluorescence quantum yield

A PBS (pH 7.4) solution of the above-described compound or labeled antibody was evaluated according to the method described in the following reference document.

"A Guide to Recording Fluorescence Quantum Yields" (a HORIBA Scientific document, available from https://www.horiba.com/fileadmin/uploads/Scientific/Documents/Fluorescence/quantumyields trad.pdf)

Regarding the fluorescence quantum yield, a high evaluation rank is highly preferable since the fluorescence quantum yield is excellent, and in the present test, it is preferable that the fluorescence quantum yield satisfies the evaluation rank "C" or higher from the viewpoint of practicality as the fluorescent compound or the labeled antibody.

### - Evaluation standards for fluorescence quantum yield -

S: 0.7 or more
A: 0.6 or more and less than 0.7
B: 0.4 or more and less than 0.6
C: 0.3 or more and less than 0.4
D: less than 0.3

**[Table 1]**

| No. | Compound or labeled antibody | Molar absorption coefficient | Fluorescence quantum yield | Binding site to biological substance |
|---|---|---|---|---|
| 111 | Compound (1-1) | A | A | Present |
| 112 | Compound (1-2) | A | A | Present |
| 113 | Compound (1-3) | A | A | Present |
| 114 | Compound (1-4) | A | S | Present |
| 115 | Compound (1-5) | A | S | Present |
| 116 | Antibody labeled with compound (1-5) | - | B | - |
| 211 | Compound (2-1) | S | B | Present |
| 212 | Compound (2-2) | S | B | Present |
| c11 | Comparative compound (1) | A | B | Absent |
| c21 | Comparative compound (2) | D | C | Present |

### (Note in table)

The antibodies labeled with the compounds (1-1) to (1-5), (2-1), and (2-2), the comparative compounds (1) and (2), and the compound (1-5) are respectively as described above.

"-" in the column of the molar absorption coefficient of the antibody labeled with the compound (1-5) means that the molar absorption coefficient is not measured.

In the column of the binding site to the biological substance, a case where the binding site to the biological substance is present is described as "Present", a case where the binding site to the biological substance is not present is described as "Absent", and a case of the labeled antibody is described as "-".

### <Example 2> Evaluation of light resistance of compound and labeled antibody

The light resistance of each of the compounds shown above or the antibody labeled therewith was evaluated as follows.

### [1] Evaluation of light resistance in aqueous solution

The above compound or labeled antibody (hereinafter, referred to as a sample) was dissolved in PBS having a pH of 7.4 so that the absorbance at the absorption wavelength peak was 0.095 to 0.105. In a state of being exposed to light using a merry-go-round type light irradiator, the obtained solution was subjected to the temporal measurement of the absorbance of the absorption wavelength peak of the sample with a spectroscope (manufactured by Hewlett-Packard Company, product name: Agilent 8453).

For the light exposure using the merry-go-round type light irradiator, a xenon lamp UXL-500D-O (product name) manufactured by Ushio, Inc. was used, and an HA-50 filter and a Y44 filter manufactured by HOYA were used as sharp cut filters (both are product names), where the light exposure intensity was 22 mW/cm² (in terms of 500 nm conversion).

The absorbance at the absorption peak wavelength before light exposure was set to 100%, and the light exposure time until the absorbance at this absorption peak wavelength decreased by 50% (the absorbance at the absorption peak wavelength reached 50%) was determined. Using the result of the exposure time of the antibody labeled with the comparative compound (1) or (2) or the comparative compound (2) as the reference value, the ratio to this reference value (the exposure time of the compound or the labeled antibody/the reference value) was calculated, and evaluation was carried out based on the following evaluation standards. A high evaluation rank is highly preferable since stability is kept for a long time.

### - Evaluation standards for light resistance -

S: 10 times or more with respect to the reference value
A: 5 times or more and less than 10 times with respect to the reference value
B: 3 times or more and less than 5 times with respect to the reference value
C: 1.2 times or more and less than 3 times with respect to the reference value
D: 1.0 times or more and less than 1.2 times with respect to the reference value
E: Less than 1.0 times with respect to the reference value

**[Table 2]**

| No. | Compound or labeled antibody | Light resistance |
|---|---|---|
| 311 | Compound (1-1) | S |
| 312 | Compound (1-2) | S |
| 313 | Compound (1-3) | C |
| 314 | Compound (1-4) | S |
| 315 | Compound (1-5) | S |
| 411 | Compound (2-2) | C |
| 412 | Antibody labeled with compound (2-2) | C |
| c31 | Comparative compound (1) | Reference values in Nos. 311 to 315 |
| c41 | Comparative compound (2) | Reference value in No. 411 |
| c42 | Antibody labeled with comparative compound (2) | Reference value in No. 412 |

From the results in Table 1 and Table 2, the following facts can be seen.

Regarding the compound having a structural moiety consisting of a dipyrromethene boron complex dye which is represented by General Formula (6) as FL and in which R⁶³ and R⁶⁶ have an alkyl group or an aryl group, the comparative compound (1) which does not have a specific phenyl group having the substituents R¹¹ to R¹⁵ defined in the present invention is inferior in light resistance. On the other hand, all of the compounds (1-1) to (1-5) which have a specific phenyl group having the substituents R¹¹ to R¹⁵ defined in the present invention, exhibit an excellent light resistance of 1.2 times or more with respect to that of the comparative compound (1), and moreover, have high water solubility required for fluorescence labelling, exhibit a molar absorption coefficient comparable to that of the comparative compound (1), exhibit a high fluorescence quantum yield, and are excellent in fluorescence intensity (brightness).

Regarding the compound having a structural moiety consisting of a dipyrromethene boron complex dye which is represented by General Formula (7) as FL, the comparative compound (2) which does not have a specific phenyl group having the substituents R¹¹ to R¹⁵ defined in the present invention is inferior in molar absorption coefficient. On the other hand, all of the compounds (2-1) and (2-2) which have a specific phenyl group having the substituents R¹¹ to R¹⁵ defined in the present invention are excellent in light absorption coefficient and fluorescence quantum yield as compared with comparative compound (2). Moreover, regarding the compound (2-2), both the compound itself and the obtained labeled biological substance are excellent in light resistance.

The present invention has been described together with the embodiments of the present invention. However, the inventors of the present invention do not intend to limit the present invention in any part of the details of the description unless otherwise specified, and it is conceived that the present invention should be broadly construed without departing from the spirit and scope of the invention shown in the attached "WHAT IS CLAIMED IS".

This application claims priority based on JP2021-133510 filed in Japan on August 18, 2021, which is incorporated herein by reference as a part of the description of the present specification.

## Claims

1. A fluorescent compound represented by General Formula (1), 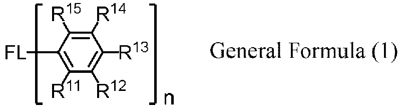
in the formula, FL indicates a phosphor moiety and n is an integer of 1 or more,
R¹¹ to R¹⁵ represent a hydrogen atom or a substituent,
provided that at least one combination of a combination of R¹¹ and R¹² or a combination of R¹² and R¹³ is a combination in which one is -SO₃⁻X⁺ and the other is a substituent, and
X⁺ represents a hydrogen ion or a metal ion.

2. The fluorescent compound according to claim 1,
wherein the at least one combination of the combination of R¹¹ and R¹² or the combination of R¹² and R¹³ is a combination in which one is -SO₃⁻X⁺ and the other is a hydroxy group, an alkoxy group, an aryloxy group, or a heteroaryloxy group.

3. The fluorescent compound according to claim 1 or 2,
wherein the fluorescent compound is represented by General Formula (2), 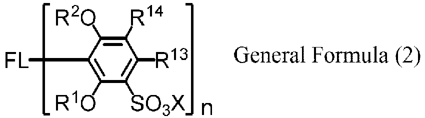
in the formula, R¹ and R² represent a hydrogen atom, an alkyl group, an aryl group, or a heteroaryl group, and
FL, R¹³, R¹⁴, X⁺, and n respectively have the same meanings as FL, R¹³, R¹⁴, X⁺, and n described above.

4. The fluorescent compound according to claim 3,
wherein R¹ and R² described above are an alkyl group, an aryl group, or a heteroaryl group.

5. The fluorescent compound according to claim 3 or 4,
wherein R¹ and R² described above are an alkyl group having an alkylene glycol structure.

6. The fluorescent compound according to any one of claims 1 to 5,
wherein the FL is a structural moiety consisting of a dipyrromethene dye, a pyrrolopyrrole dye, a pyrene dye, a coumarin dye, or a cyanine dye.

7. The fluorescent compound according to claim 6,
wherein the FL is a structural moiety consisting of a dipyrromethene boron complex dye.

8. The fluorescent compound according to claim 7,
wherein the dipyrromethene boron complex dye is represented by General Formula (6), 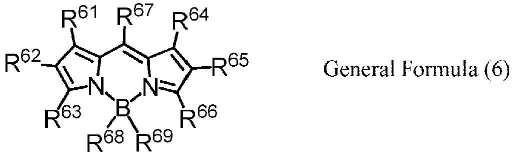
in the formula, R⁶¹ to R⁶⁷ represent a hydrogen atom or a substituent,
R⁶⁸ and R⁶⁹ represent a hydrogen atom, a hydroxy group, a halogen atom, an alkoxy group, an alkyl group, an aryl group, -C≡CR^{b}, or a cyano group,
R^{b} represents a hydrogen atom, an alkyl group, or an aryl group, and
in a dye represented by the formula, n pieces of hydrogen atoms are removed from any one of R⁶¹ to R⁶⁹ so that the FL is n-valent.

9. The fluorescent compound according to claim 8,
wherein R⁶³ and R⁶⁶ described above are an alkyl group or an aryl group.

10. The fluorescent compound according to claim 8,
wherein the dipyrromethene boron complex dye is represented by General Formula (7), 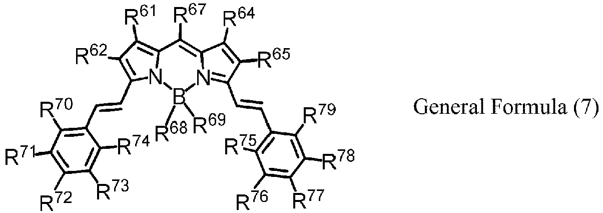
in the formula, R⁶¹, R⁶², R⁶⁴, R⁶⁵, and R⁶⁷ to R⁶⁹ respectively have the same meanings as R⁶¹, R⁶², R⁶⁴, R⁶⁵, and R⁶⁷ to R⁶⁹ described above,
R⁷⁰ to R⁷⁹ represent a hydrogen atom or a substituent, and
in a dye represented by the formula, n pieces of hydrogen atoms are removed from any one of R⁶¹, R⁶², R⁶⁴, R⁶⁵, R⁶⁷ to R⁶⁹, or R⁷⁰ to R⁷⁹ so that the FL is n-valent.

11. A fluorescently labeled biological substance that is obtained by bonding the fluorescent compound according to any one of claims 1 to 10 to a biological substance.

12. The fluorescently labeled biological substance according to claim 11,
wherein the biological substance is any one of a protein, an amino acid, a nucleic acid, a sugar chain, or a lipid.

13. The fluorescently labeled biological substance according to claim 11 or 12,
wherein the bonding of the fluorescent compound to the biological substance is a bonding formed by any one of the following i) to v),
i) a non-covalent bond or covalent bond between peptides,
ii) Van der Waals interaction between a long-chain alkyl group in a compound and a lipid bilayer or lipid in a biological substance,
iii) an amide bond formed by reacting an N-hydroxysuccinimide ester in a compound with an amino group in a biological substance,
iv) a thioether bond formed by reacting a maleimide group in a compound with a sulfanyl group in a biological substance, and
v) a bond associated with a formation of a triazole ring, which is formed by a Click reaction between an azide group in a compound and an acetylene group in a biological substance, or between an acetylene group in a compound and an azide group in a biological substance.
